# EUROPEAN PATENT SPECIFICATION

(11) **EP 3 583 093 B1**
(45) Date of publication and mention of the grant of the patent: **14.04.2021**
(21) Application number: 18703609.0
(22) Date of filing: 13.02.2018
(51) Int. Cl.: C07D 231/14, C07F 9/50, C07F 9/54, C07C 201/12, C07C 205/19, C07D 241/24, C07F 5/02

(54) **PROCESS FOR PREPARING SUBSTITUTED BIPHENYLS**
VERFAHREN ZUR HERSTELLUNG SUBSTITUIERTER BIPHENYLE
PROCÉDÉ DE PRÉPARATION DE BIPHÉNYLES SUBSTITUÉS

(30) Priority: 14.02.2017 EP 17156124
(43) Date of publication of application: 25.12.2019
(73) Proprietor: BASF SE, 67056 Ludwigshafen am Rhein (DE)
(72) Inventor: FRASSETTO, Timo, 67056 Ludwigshafen (DE); MAYER, Horst, 67056 Ludwigshafen (DE); KRAEMER, Siegfried, 67056 Ludwigshafen (DE)
(86) International application number: PCT/EP2018/053522
(87) International publication number: WO 2018/149813

(56) References cited:
- WO-A1-2015/011032
- FEI-CHEN GUO ET AL: "N,N,N',N'-tetra(diphenylphosphinomethyl)p yridine-2,6-diamine/palladium catalyzed Suzuki-Miyaura coupling of aryl and heteroaryl halides", CATALYSIS COMMUNICATIONS, vol. 66, 1 June 2015 (2015-06-01), pages 87-90, XP055421759, AMSTERDAM, NL ISSN: 1566-7367, DOI: 10.1016/j.catcom.2015.03.028 cited in the application
- NICOLAS FLEURY-BRÉGEOT ET AL: "Suzuki-Miyaura Cross-Coupling of Potassium Dioxolanylethyltrifluoroborate and Aryl/Heteroaryl Chlorides", ORGANIC LETTERS , 14(23), 6012-6015 CODEN: ORLEF7; ISSN: 1523-7052, vol. 15, no. 7, 5 April 2013 (2013-04-05), pages 1536-1539, XP055421749, ISSN: 1523-7060, DOI: 10.1021/ol400320q

## Description

The present invention relates to a process for preparing substituted 2-nitrobiphenyls via Suzuki coupling using a palladium catalyst with specific phosphorus ligands and a solvent mixture containing water and an organic solvent which is at least partially miscible with water.

Functionalized biphenyl compounds are of great interest especially as pharmaceuticals and pesticides, and as precursors of such active ingredients. For instance, 2-nitro and 2-aminobiphenyls are important precursors for aryl- and heteroarylcarboxamides which find use as fungicides, and for which boscalid, fluxapyroxad, bixafen or pyraziflumid are prominent representatives. For their synthesis, a series of organometallic methods is available, which offer efficient access to a multitude of biphenyl derivatives. The most frequently applied is the Suzuki coupling.

The Suzuki coupling (also called Suzuki-Miyaura coupling or Suzuki reaction or Suzuki-Miyaura reaction) is a cross coupling reaction in which an organoboron compound is reacted with an organic halogenide or sulfonate in the presence of a transition metal catalyst, mostly a Pd or Ni catalyst, and in general also of a base.

Principally, the known processes for preparing nitro- or aminobiphenyls via Suzuki coupling work well, at least on a laboratory scale. However, there is still room for improvement, especially with respect to an application in large-scale industrial processes. For instance, the amount of required Pd in the catalyst is still rather high.

WO 2015/011032 relates to a process for preparing chlorinated biphenylanilines or anilides by Suzuki coupling using a palladium catalyst containing an optionally substituted di-tert-butylphenyl phosphine or a salt thereof as ligand. This catalyst is said to avoid the undesired formation of triphenyl compounds. In the halide starting compound II the leaving group Hal is Br or I. In the examples the coupling reaction is carried out in a mixture of water and 1-butanol as solvent in the presence of potassium carbonate as base. The Pd catalyst is used in an amount of 0.12 mol%, calculated on the basis of the Pd content and relative to 1 mol of the halide.

F.-C. Guo et al. mention in Catalysis Communications 2015, 66, 87-90 a similar transformation.

Although in this process the amount of Pd is already reduced as compared to older processes, there is still room for improvement. Moreover, the use of aromatic bromides or iodides is not desirable, not only because of their cost, especially in case of the iodide, but also because of environmental concerns connected with bromide or iodide-containing waste water.

It was thus an object of the present invention to provide a process for producing nitro-substituted biphenyls via Suzuki coupling which avoids some of the drawbacks of the prior art processes, especially when these are applied on a large scale. Especially it was the object of the present invention to provide a process for producing nitro-substituted biphenyls via Suzuki coupling which requires distinctly lower amounts of palladium, suppresses homocoupling, avoids bromide- and iodide-containing waste water and is well-suited for large-scale applications.

The object is achieved by a process for preparing substituted biphenyls of the formula I 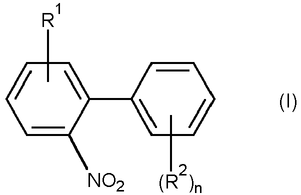 in which the substituents are each defined as follows:
- R¹: is hydrogen, cyano, F, C₁-C₄-alkyl, C₁-C₄-haloalkyl, C₁-C₄-alkoxy or C₁-C₄-haloalkoxy;
- R²: is cyano, nitro, F, Cl, C₁-C₄-alkyl, C₁-C₄-haloalkyl, C₃-C₁₀-cycloalkyl which may carry 1, 2, 3 or 4 C₁-C₄-alkyl substituents; C₃-C₁₀-halocycloalkyl, C₁-C₆-alkoxy, C₁-C₆-haloalkoxy, C₁-C₆-alkylcarbonyl, C₁-C₆-haloalkylcarbonyl, C₁-C₆-alkoxycarbonyl, or C₁-C₆-haloalkoxycarbonyl; and
- n: is 0, 1, 2 or 3, where, in case that n is 2 or 3, the R² radicals may have identical or different definitions;
which comprises reacting a compound of the formula II 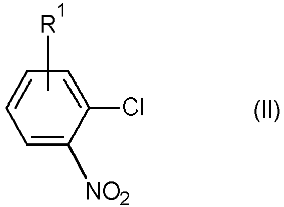 in which R¹ is as defined above, in the presence of a base and of a palladium catalyst, where the palladium catalyst is introduced into the reaction in the form of
- a palladium source and a phosphorus ligand of the formula III or a salt thereof 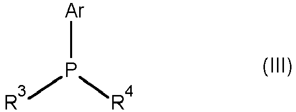 in which
   - Ar: is a C₆-C₁₀-aryl radical or a 5- or 6-membered heteroaryl ring containing 1, 2, 3 or 4 heteroatoms selected from the group consisting of N and O as ring members, where the aryl radical and the heteroaryl ring are optionally substituted with 1, 2 or 3 substituents independently selected from the group consisting of C₁-C₆-alkyl, C₁-C₆-alkoxy, trifluoromethyl and phenyl;
   - R³: is C₁-C₈-alkyl or C₃-C₁₀-cycloalkyl; and
   - R⁴: is C₁-C₈-alkyl or C₃-C₁₀-cycloalkyl;
or
- a palladium complex containing at least one phosphorus ligand of the formula III as defined above or a salt thereof;
in a solvent mixture of water and an organic solvent which is at least partially miscible with water,
with an organoboron compound of the formula IV 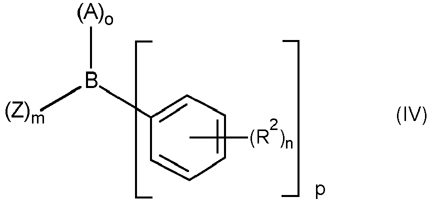 wherein R² and n are as defined above and the compound of formula IV is selected from the group consisting of
(i) boronic acids with o = 0, m = 2; p = 1 and Z = hydroxyl groups, or their trimers;
(ii) boronic acid derivates with o = 0, m = 2; p = 1 and Z = halogen; C₁-C₄-alkyl, C₁-C₆-alkoxy or C₆-C₁₀-aryloxy;
(iii) borinic acids or borinic acid derivatives with o = 0, m = 1; p = 2 and Z = hydroxy, halogen, C₁-C₄-alkyl, C₁-C₆-alkoxy or C₆-C₁₀-aryloxy;
(iv) mixed borinic acids or borinic acid derivatives with o = 1, m = 1; p = 1, A = C₁-C₄-alkyl and Z = hydroxy, halogen, C₁-C₄-alkyl, C₁-C₆-alkoxy or C₆-C₁₀-aryloxy;
(v) cyclic boronic esters with o = 0, m = 2 and p = 1, wherein the two Z groups form together a bridging group -O-(CH₂)_{q}-O-, wherein q is 2 or 3, so that the two Z groups, together with the boron atom to which they are attached, form a 5- or 6-membered ring, where the CH₂ groups are optionally substituted by one or two C₁-C₄-alkyl groups;
(vi) boronates with o = 0, m = 3, p = 1 and Z = hydroxyl, halogen, C₁-C₄-alkyl, C₁-C₆-alkoxy or C₆-C₁₀-aryloxy, and accompanied by a cation which compensates the negative charge of the boronate anion;
(vii) triarylboranes with o = 0, m = 0 and p = 3;
(viii) tetraarylborates with o = 0, m = 0 and p = 4, and accompanied by a cation which compensates the negative charge of the borate anion;
where the reaction is carried out at a temperature of from 80 to 140°C.

The organic moieties mentioned in the above definitions of the variables are - like the term halogen - collective terms for individual listings of the individual group members. The prefix Cₙ-Cₘ indicates in each case the possible number of carbon atoms in the group.

The term halogen denotes in each case fluorine, bromine, chlorine or iodine, in particular fluorine, chlorine or bromine.

The term "alkyl" as used herein and in the alkyl moieties of alkoxy, alkylcarbonyl, alkoxycarbonyl and the like refers to saturated straight-chain or branched hydrocarbon radicals having 1 to 2 ("C₁-C₂-alkyl"), 1 to 3 ("C₁-C₃-alkyl"),1 to 4 ("C₁-C₄-alkyl"), 1 to 6 ("C₁-C₆-alkyl") or 1 to 8 ("C₁-C₈-alkyl") carbon atoms. C₁-C₂-Alkyl is methyl or ethyl. C₁-C₃-Alkyl is additionally propyl and isopropyl. C₁-C₄-Alkyl is additionally n-butyl, 1-methylpropyl (sec-butyl), 2-methylpropyl (isobutyl) or 1,1-dimethylethyl (tert-butyl). C₁-C₆-Alkyl is additionally also, for example, pentyl, 1-methylbutyl, 2-methylbutyl, 3-methylbutyl, 2,2-dimethylpropyl, 1-ethylpropyl, 1,1-dimethylpropyl, 1,2-dimethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, or 1-ethyl-2-methylpropyl. C₁-C₈-Alkyl is additionally also, for example, heptyl, octyl and the isomers thereof.

The term "haloalkyl" as used herein, which is also expressed as "alkyl which is partially or fully halogenated", refers to straight-chain or branched alkyl groups having 1 to 2 ("C₁-C₂-haloalkyl"), 1 to 3 ("C₁-C₃-haloalkyl"), 1 to 4 ("C₁-C₄-haloalkyl") or 1 to 6 ("C₁-C₆-haloalkyl") carbon atoms (as mentioned above), where some or all of the hydrogen atoms in these groups are replaced by halogen atoms as mentioned above: in particular C₁-C₂-haloalkyl, such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl or pentafluoroethyl. C₁-C₃-haloalkyl is additionally, for example, 1-fluoropropyl, 2-fluoropropyl, 3-fluoropropyl, 1,1-difluoropropyl, 2,2-difluoropropyl, 1,2-difluoropropyl, 3,3-difluoropropyl, 3,3,3-trifluoropropyl, heptafluoropropyl, 1,1,1-trifluoroprop-2-yl, 3-chloropropyl and the like. Examples for C₁-C₄-haloalkyl are, apart those mentioned for C₁-C₃-haloalkyl, 4-chlorobutyl and the like.

"Methyl which is substituted by 1, 2 or 3 fluorine atoms" is fluoromethyl, difluoromethyl or trifluoromethyl.

"C₁-C₆-Hydroxyalkyl" is C₁-C₆-alkyl, as defined above, where one hydrogen atom is replaced by a hydroxy group. Examples are hydroxymethyl, 1-hydroxyethyl, 2-hydroxyethyl, 1-hydroxypropyl, 2-hydroxypropyl, 3-hydroxypropyl, 1-hydroxy-1-methylethyl, 2-hydroxy-1-methylethyl, 1-hydroxybutyl, 2-hydroxybutyl, 3-hydroxybutyl, 4-hydroxybutyl, 1-hydroxypentyl, 2-hydroxypentyl, 3-hydroxypentyl, 4-hydroxypentyl, 5-hydroxypentyl, 1-hydroxyhexyl, 2-hydroxyhexyl, 3-hydroxyhexyl, 4-hydroxyhexyl, 5-hydroxyhexyl, 6-hydroxyhexyl, and the like.

The term "cycloalkyl" as used herein refers to mono- or bicyclic saturated hydrocarbon radicals having 3 to 10 ("C₃-C₁₀-cycloalkyl"), 3 to 8 ("C₃-C₈-cycloalkyl"), in particular 3 to 6 ("C₃-C₆-cycloalkyl") or 3 to 5 ("C₃-C₅-cycloalkyl") or 3 to 4 ("C₃-C₄-cycloalkyl") carbon atoms. Examples of monocyclic radicals having 3 to 4 carbon atoms are cyclopropyl and cyclobutyl. Examples of monocyclic radicals having 3 to 5 carbon atoms are cyclopropyl, cyclobutyl and cyclopentyl. Examples of monocyclic radicals having 3 to 6 carbon atoms are cyclopropyl, cyclobutyl, cyclopentyl and cyclohexyl. Examples of monocyclic radicals having 3 to 8 carbon atoms are cyclopropyl, cyclobutyl, cyclopentyl, cyclohexyl, cycloheptyl and cyclooctyl. Examples of monocyclic radicals having 3 to 10 carbon atoms are cyclopropyl, cyclobutyl, cyclopentyl, cyclohexyl, cycloheptyl, cyclooctyl, cyclononyl and cyclodecyl. The bicyclic radicals can be condensed or bridged rings. Examples of bicyclic condensed radicals having 6 to 10 carbon atoms comprise bicyclo[3.1.0]hexyl, bicyclo[3.2.0]heptyl, bicyclo[3.3.0]octyl (1,2,3,3a,4,5,6,6a-octahydropentalenyl), bicyclo[4.2.0]octyl, bicyclo[4.3.0]nonyl (2,3,3a,4,5,6,7,7a-octahydro-1H-indene), bicyclo[4.4.0]decyl (decalinyl) and the like. Examples of bridged bicyclic condensed radicals having 7 to 10 carbon atoms comprise bicyclo[2.2.1]heptyl, bicyclo[3.1.1]heptyl, bicyclo[2.2.2]octyl, bicyclo[3.2.1]octyl and the like. Preferably, the term cycloalkyl denotes a monocyclic saturated hydrocarbon radical.

The term "halocycloalkyl" as used herein, which is also expressed as "cycloalkyl which is partially or fully halogenated", refers to mono- or bicyclic saturated hydrocarbon groups having 3 to 10 ("C₃-C₁₀-halocycloalkyl") or 3 to 8 ("C₃-C₈-halocycloalkyl") or preferably 3 to 6 ("C₃-C₆-halocycloalkyl") or 3 to 5 ("C₃-C₅-halocycloalkyl") or 3 to 4 ("C₃-C₄-halocycloalkyl") carbon ring members (as mentioned above) in which some or all of the hydrogen atoms are replaced by halogen atoms as mentioned above, in particular fluorine, chlorine and bromine.

"Alkoxy" is an alkyl group attached via an oxygen atom. The term "C₁-C₂-alkoxy" is a C₁-C₂-alkyl group, as defined above, attached via an oxygen atom. The term "C₁-C₃-alkoxy" is a C₁-C₃-alkyl group, as defined above, attached via an oxygen atom. The term "C₁-C₄-alkoxy" is a C₁-C₄-alkyl group, as defined above, attached via an oxygen atom. The term "C₁-C₆-alkoxy" is a C₁-C₆-alkyl group, as defined above, attached via an oxygen atom. C₁-C₂-Alkoxy is methoxy or ethoxy. C₁-C₃-Alkoxy is additionally, for example, n-propoxy and 1-methylethoxy (isopropoxy). C₁-C₄-Alkoxy is additionally, for example, butoxy, 1-methylpropoxy (sec-butoxy), 2-methylpropoxy (isobutoxy) or 1,1-dimethylethoxy (tert-butoxy). C₁-C₆-Alkoxy is additionally, for example, pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy or 1-ethyl-2-methylpropoxy.

"Haloalkoxy" is a haloalkyl group attached via an oxygen atom. The term "C₁-C₂-haloalkoxy" is a C₁-C₂-haloalkyl group, as defined above, attached via an oxygen atom. The term "C₁-C₃-haloalkoxy" is a C₁-C₃-haloalkyl group, as defined above, attached via an oxygen atom. The term "C₁-C₄-haloalkoxy" is a C₁-C₄-haloalkyl group, as defined above, attached via an oxygen atom. The term "C₁-C₆-haloalkoxy" is a C₁-C₆-haloalkyl group, as defined above, attached via an oxygen atom. C₁-C₂-Haloalkoxy is, for example, OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy or OC₂F₅. C₁-C₃-Haloalkoxy is additionally, for example, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 2-bromopropoxy, 3-bromopropoxy, 3,3,3-trifluoropropoxy, 3,3,3-trichloropropoxy, OCH₂-C₂F₅, OCF₂-C₂F₅, 1-(CH₂F)-2-fluoroethoxy, 1-(CH₂Cl)-2-chloroethoxy or 1-(CH₂Br)-2-bromoethoxy. C₁-C₄-Haloalkoxy is additionally, for example, 4-fluorobutoxy, 4-chlorobutoxy, 4-bromobutoxy or nonafluorobutoxy. C₁-C₆-Haloalkoxy is additionally, for example, 5-fluoropentoxy, 5-chloropentoxy, 5-brompentoxy, 5-iodopentoxy, undecafluoropentoxy, 6-fluorohexoxy, 6-chlorohexoxy, 6-bromohexoxy, 6-iodohexoxy or dodecafluorohexoxy.

The term "alkylcarbonyl" is a C₁-C₆-alkyl ("C₁-C₆-alkylcarbonyl"), preferably a C₁-C₄-alkyl ("C₁-C₄-alkylcarbonyl") group, as defined above, attached via a carbonyl [C(=O)] group. Examples are acetyl (methylcarbonyl), propionyl (ethylcarbonyl), propylcarbonyl, isopropylcarbonyl, n-butylcarbonyl and the like.

The term "haloalkylcarbonyl" is a C₁-C₆-haloalkyl ("C₁-C₆-haloalkylcarbonyl"), preferably a C₁-C₄-haloalkyl ("C₁-C₄-haloalkylcarbonyl") group, as defined above, attached via a carbonyl [C(=O)] group. Examples are trifluoromethylcarbonyl, 2,2,2-trifluoroethylcarbonyl and the like.

The term "alkoxycarbonyl" is a C₁-C₆-alkoxy ("C₁-C₆-alkoxycarbonyl"), preferably a C₁-C₄-alkoxy ("C₁-C₄-alkoxycarbonyl") group, as defined above, attached via a carbonyl [C(=O)] group. Examples are methoxycarbonyl, ethoxycarbonyl, propoxycarbonyl, isopropoxycarbonyl, n-butoxycarbonyl and the like.

The term "haloalkoxycarbonyl" is a C₁-C₆-haloalkoxy ("C₁-C₆-haloalkoxycarbonyl"), preferably a C₁-C₄-haloalkoxy ("C₁-C₄-haloalkoxycarbonyl") group, as defined above, attached via a carbonyl [C(=O)] group. Examples are trifluoromethoxycarbonyl, 2,2,2-trifluoroethoxycarbonyl and the like.

If the term "aryl" as used herein and in the aryl moieties of aryloxy is used without prefix (Cₙ-Cₘ), it indicates an aryl group with 6 to 30, in particular 6 to 14, specifically 6 to 10 carbon atoms as ring members. Aryl is a mono-, bi- or polycyclic carbocyclic (i.e. without heteroatoms as ring members) aromatic radical. One example for a monocyclic aromatic radical is phenyl. In bicyclic aryl rings two aromatic rings are condensed, i.e. they share two vicinal C atoms as ring members. One example for a bicyclic aromatic radical is naphthyl. In polycyclic aryl rings, three or more rings are condensed. Examples for polycyclic aryl radicals are phenanthrenyl, anthracenyl, tetracenyl, 1H-benzo[a]phenalenyl, pyrenyl and the like.

"C₆-C₁₀-Aryl" is phenyl, 1-naphthyl or 2-naphthyl.

"Aryloxy" is aryl, as defined above, bound via an oxygen atom to the remainder of the molecule.

"C₆-C₁₀-Aryloxy" is phenoxy, 1-naphthyloxy or 2-naphthyloxy.

5- or 6-membered heteroaryl rings containing 1, 2, 3 or 4 heteroatoms selected from the group consisting of N and O as ring members are monocyclic heteroaromatic rings. In the 6-membered heteroaryl rings the heteroatom ring members can only be nitrogen atoms. Examples for 5- or 6-membered heteroaromatic rings containing 1, 2, 3 or 4 heteroatoms selected from N and O as ring members are 2-furyl, 3-furyl, 1-pyrrolyl, 2-pyrrolyl, 3-pyrrolyl, 1-pyrazolyl, 3-pyrazolyl, 4-pyrazolyl, 5-pyrazolyl, 1-imidazolyl, 2-imidazolyl, 4-imidazolyl, 5-imidazolyl, 2-oxazolyl, 4-oxazolyl, 5-oxazolyl, 3-isoxazolyl, 4-isoxazolyl, 5-isoxazolyl, 1,3,4-triazol-1-yl, 1,3,4-triazol-2-yl, 1,3,4-triazol-3-yl, 1,2,3-triazol-1-yl, 1,2,3-triazol-2-yl, 1,2,3-triazol-4-yl, 1,2,5-oxadiazol-3-yl, 1,2,3-oxadiazol-4-yl, 1,2,3-oxadiazol-5-yl, 1,3,4-oxadiazol-2-yl, tetrazol-1-yl, tetrazol-2-yl, tetrazol-5-yl, 2-pyridinyl, 3-pyridinyl, 4-pyridinyl, 1-oxopyridin-2-yl, 1-oxopyridin-3-yl, 1-oxopyridin-4-yl, 3-pyridazinyl, 4-pyridazinyl, 2-pyrimidinyl, 4-pyrimidinyl, 5-pyrimidinyl, 2-pyrazinyl, 1,3,5-triazin-2-yl, 1,2,4-triazin-3-yl, 1,2,4-triazin-5-yl, 1,2,3,4-tetrazin-1-yl, 1,2,3,4-tetrazin-2-yl, 1,2,3,4-tetrazin-5-yl and the like.

The remarks made below regarding preferred embodiments of the process according to the invention, especially regarding preferred embodiments of the radicals of the different reactants and products (to be more precise preferred embodiments of the variables of the compounds of formulae I, II, III, IV, V and VI, especially with respect to their substituents R¹, R², R³, R⁴, R⁵, Ar and n) and of the reaction conditions of the processes according to the invention, apply either taken alone or, more particularly, in any conceivable combination with one another.

The remarks to preferred embodiments of R¹ apply both to formula I as well as to formula II, V and VI, unless explicitly specified otherwise. The remarks to preferred embodiments of R² and n apply both to formula I as well as to formula IV, V and VI, unless explicitly specified otherwise.

In a particular embodiment, R¹ is hydrogen or fluorine. Specifically, R¹ is hydrogen.

In a specific embodiment, R¹ is in para position to the nitro group (this definition of the position of R¹ is of course only relevant if R¹ is not hydrogen) . In case of the biphenyl compounds I, nitro group in this case refers of course to the nitro group on the same phenyl ring as R¹ (and not to a possible nitro group R²).

In particular, R² is F or Cl.

Preferably, n is 1, 2 or 3; specifically 1 or 3.

More particularly, R² is F or Cl and n is 1, 2 or 3. Specifically, R² is F or Cl and n is 1 or 3.

In a particular embodiment, the biphenyl compound I is 4-chloro-2'-nitro-biphenyl, 3,4-dichloro-2'-nitro-biphenyl, 3,4-difluoro-2'-nitro-biphenyl, 3,4,5-trifluoro-2'-nitro-biphenyl, 3-chloro-4,5-difluoro-2'-nitro-biphenyl, 3,4-dichloro-5'-fluoro-2'-nitro-biphenyl or 3,5-dichloro-4-fluoro-2'-nitro-biphenyl. Specifically, the biphenyl compound I is 4-chloro-2'-nitro-biphenyl, 3,4-dichloro-2'-nitro-biphenyl, 3,4,5-trifluoro-2'-nitro-biphenyl or 3,4-dichloro-5'-fluoro-2'-nitro-biphenyl; very specifically 4-chloro-2'-nitro-biphenyl or 3,4,5-trifluoro-2'-nitro-biphenyl.

The organoboron compound IV as defined under (i) in which o = 0, m = 2; p = 1 and Z = OH is a boronic acid of formula IVa. Its trimer is a boroxine and has formula tri-IVa:

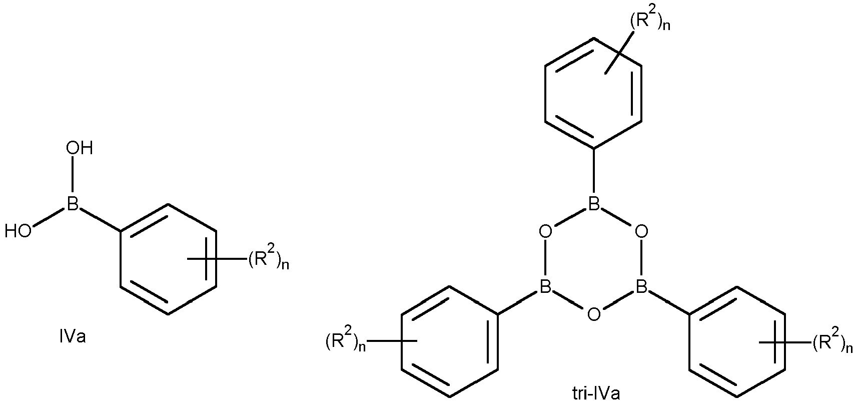

The boronic acid derivates as defined under (ii) with o = 0, m = 2; p = 1 and Z = halogen; C₁-C₄-alkyl, C₁-C₆-alkoxy or C₆-C₁₀-aryloxy are compounds of formula IVb, wherein Z = halogen; C₁-C₄-alkyl, C₁-C₆-alkoxy or C₆-C₁₀-aryloxy:

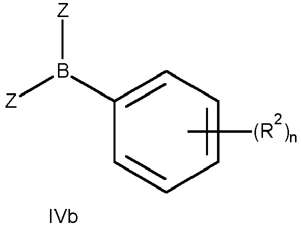

The borinic acids or borinic acid derivatives as defined under (iii) with o = 0, m = 1; p = 2 and Z = hydroxy, halogen, C₁-C₄-alkyl, C₁-C₆-alkoxy or C₆-C10-aryloxy are compounds of formula IVc (borinic acids) or compounds of formula IVd (borinic acid derivatives), wherein Z = halogen, C₁-C₄-alkyl, C₁-C₆-alkoxy or C₆-C₁₀₋aryloxy:

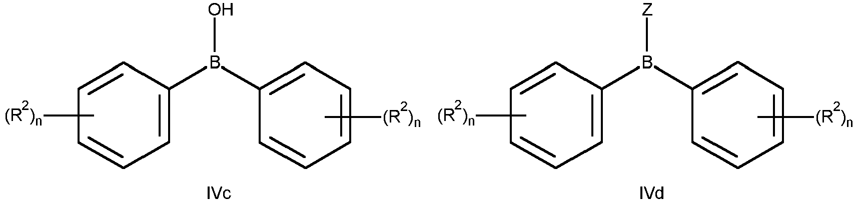

The mixed borinic acids or borinic acid derivatives as defined under (iv) with o = 1, m = 1; p = 1, A = C₁-C₄-alkyl and Z = hydroxy, halogen, C₁-C₄-alkyl, C₁-C₆-alkoxy or C₆-C₁₀-aryloxy are compounds of formula IVe (mixed borinic acids) or compounds of formula IVf (mixed borinic acid derivatives), wherein Z = halogen, C₁-C₄-alkyl, C₁-C₆-alkoxy or C₆-C₁₀-aryloxy:

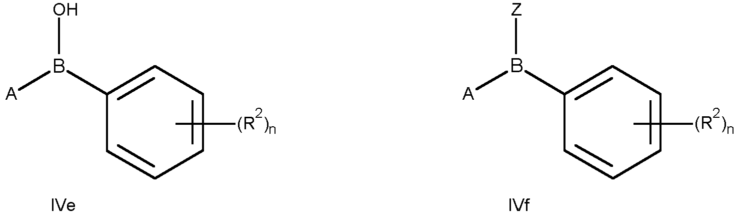

The cyclic boronic esters as defined under (v) with o = 0, m = 2 and p = 1, wherein the two Z groups form together a bridging group -O-(CH₂)_{q}-O-, wherein q is 2 or 3, so that the two Z groups, together with the boron atom to which they are attached, form a 5- or 6- membered ring, where the CH₂ groups are optionally substituted by one or two C₁-C₄-alkyl groups are compounds of formula IVg: 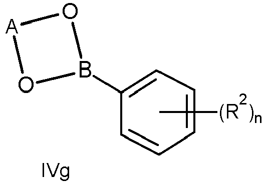 wherein A is -C(R^{A1})(R^{A2})-C(R^{A3})(R^{A4})- or -C(R^{A1})(R^{A2})-C(R^{A3})(R^{A4})-C(R^{A5})(R^{A6})-, where R^{A1}, R^{A2}, R^{A3}, R^{A4}, R^{A5} and R^{A6}, independently of each other, are hydrogen or C₁-C₄-alkyl.

The boronates as defined under (vi) with o = 0, m = 3, p = 1 and Z = hydroxyl, halogen, C₁-C₄-alkyl, C₁-C₆-alkoxy or C₆-C₁₀-aryloxy, and accompanied by a cation which compensates the negative charge of the boronate anion are compounds of formula IVh, wherein each Z is independently hydroxyl, halogen, C₁-C₄-alkyl, C₁-C₆-alkoxy or C₆-C₁₀-aryloxy and (M^{a+})_{1/a} is a cation equivalent:

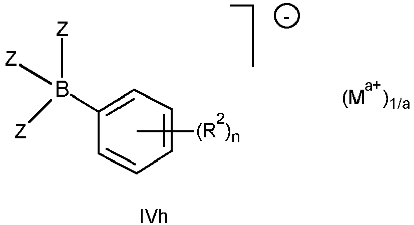

The triarylboranes as defined under (vii) with o = 0, m = 0 and p = 3 are compounds of formula IVi:

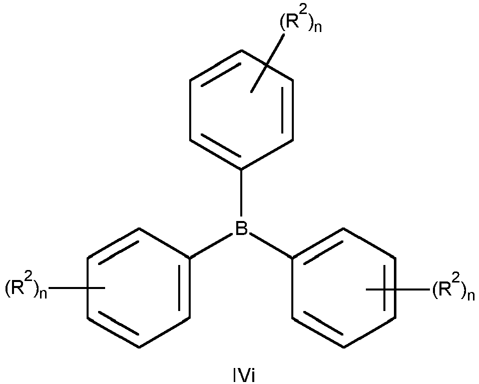

The tetraarylborates as defined under (viii) with o = 0, m = 0 and p = 4, and accompanied by a cation which compensates the negative charge of the borate anion, are compounds of formula IVj, wherein (M^{a+})_{1/a} is a cation equivalent:

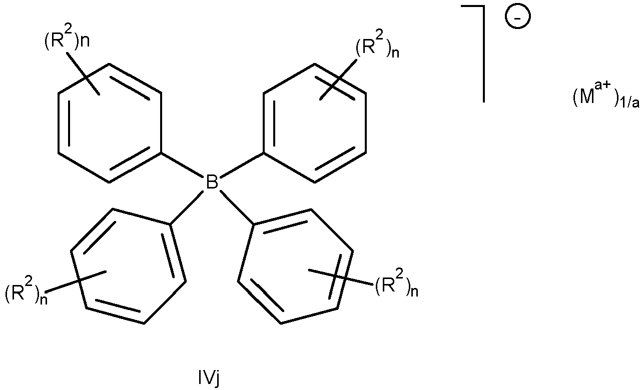

M in compounds IVh and IVj is preferably an alkali or earth alkaline metal cation or an ammonium cation (NR^{a}R^{b}R^{c}R^{d})⁺, wherein R^{a}, R^{b}, R^{c} and R^{d}, independently of each other, are hydrogen, C₁-C₆-alkyl or C₁-C₆-hydroxyalkyl. If M is an alkali metal cation or an ammonium cation, a is 1. If M is an earth alkaline metal cation, a is 2. More preferably, M is an alkali metal cation.

In the above organoboron compounds R² and n have one of the above general or, in particular, one of the above preferred meanings. In a particular embodiment, (R₂)ₙ is 4-chloro, 3,4-dichloro, 3,4-difluoro, 3,4,5-trifluoro, 3-chloro-4,5-difluoro or 3,5-dichloro-4-fluoro. Specifically, (R₂)ₙ is 4-chloro, 3,4-dichloro or 3,4,5-trifluoro. Very specifically, (R₂)ₙ is 4-chloro or 3,4,5-trifluoro.The positions relate to the 1-position of the attachment of the phenyl ring to the boron atom.

A in the mixed borinic acids or borinic acid derivatives as defined under (iv) is in particular methyl.

Preferably, the organoboron compound IV is a phenylboronic acid IVa or a diphenylborinic acid IVc 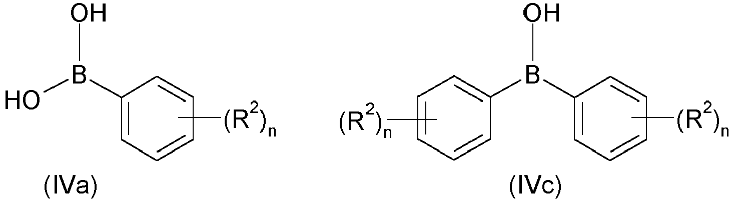 or a mixture of IVa and IVc, in which R² and n have one of the above general or, in particular, one of the above preferred meanings. In particular, the organoboron compound IV is a phenylboronic acid IVa.

In a particular embodiment, (R₂)ₙ in IVa and IVc is 4-chloro, 3,4-dichloro, 3,4-difluoro, 3,4,5-trifluoro, 3-chloro-4,5-difluoro or 3,5-dichloro-4-fluoro, more particularly 4-chloro, 3,4-dichloro or 3,4,5-trifluoro, and specifically 4-chloro or 3,4,5-trifluoro. The positions relate to the 1-position of the attachment point of the phenyl ring to the boron atom.

The organoboron compounds as defined under (i) to (viii) and methods for preparing them are known in the art and described, for example, in WO 2015/011032 and the literature cited therein.

The compounds of formulae II and IV are used in a molar ratio of preferably from 5:1 to 1:5, more preferably from 2:1 to 1:2, even more preferably from 1.5:1 to 1:1.5, in particular from 1.1:1 to 1:1.1, specifically from 1.05:1 to 1:1.05, and very specifically of approximately 1:1. "Approximately" is intended to include deviations from ideal stoichiometry caused, for example, by weight errors. Such errors are in general below 10%, mostly below 5%.

The molar ratios of compounds IV as given above relate to the number of phenyl rings contained in the organoboron molecule IV which can react in the Suzuki reaction.

Thus, consequently, the molar ratio of compounds II and IVa, IVb, IVe, IVf, IVg or IVh (having one phenyl ring per organoboron molecule which can react in the Suzuki reaction), the compounds IV here counted as such, is preferably from 5:1 to 1:5, more preferably from 2:1 to 1:2, even more preferably from 1.5:1 to 1:1.5, in particular from 1.1:1 to 1:1.1, specifically from 1.05:1 to 1:1.05, and very specifically of approximately 1:1;
the molar ratio of compounds II and IVc or IVd (having two phenyl rings per organoboron molecule which can react in the Suzuki reaction), the compounds IV here counted as such, is preferably from 10:1 to 1:2.5, more preferably 4:1 to 1:1, even more preferably from 3:1 to 1:0.75, in particular from 2.2:1 to 1:0.55, specifically from 2.1:1:1 to 1:0.53, and very specifically of approximately 2:1;
the molar artio fo compounds II and tri-IVa or IVi (having three phenyl rings per organoboron molecule which can react in the Suziki reaction), the compounds IV here counted as such, is preferably from 15:1 to 1:1.7, more preferably from 6: 1 to 1:0.7, even more preferably from 4.5:1 to 1:0.5, in particular from 3.3:1 to 1:0.37, specifically from 3.14:1 to 1:0.35, and very specifically of approximately 3:1; and the molar artio of compounds II and IVj (having four phenyl rings per organoboron molecule which can react in the Suzuki reaction), the compound IVj here counted as such, is preferably from 20:1 to 1:1.25, more preferably from 8:1 to 1:0.5, even more preferably from 6:1 to 1:0.38, in particular from 4.4:1 to 1:0.28, specifically from 4.2:1 to 1:0.26, and very specifically of approximately 4:1.

As however the removal of the halogen compound II from the reaction mixture after completion of the reaction is sometimes more difficult than the removal of the organoboron compound IV, it may be advantageous to use the organoboron compound IV in at least equimolar amounts, better in slight excess, so that the halogen compound II is reacted more or less completely. In this case, compounds of formulae II and IV (the latter counted as the number of phenyl rings contained in the organoboron molecule IV which can react in the Suzuki reaction) are used in a molar ratio of preferably from 1:1 to 1:1.5, more preferably from 1:1 to 1:1.1, in particular from 1:1 to 1:1.05 and specifically from 1:1.01 to 1:1.05. However, the inverse stoichiometry is also possible; i.e. compound II can also be used in slight excess; meaning that compounds of formulae II and IV (the latter counted as the number of phenyl rings contained in the organoboron molecule IV which can react in the Suzuki reaction) are used in a molar ratio of preferably from 1:1 to 1.5:1, more preferably from 1:1 to 1.1:1, in particular from 1:1 to 1.05:1 and specifically from 1.01:1 to 1.05:1.

Phenyl rings contained in compound IV which can react in the Suzuki reaction are those phenyl rings which are directly bound to the boron atom. Thus, phenyl rings contained in Z, if this is aryloxy, are not counted.

In case of compounds IV, "equimolar amounts" and "excess" amounts are of course related to the number of phenyl rings contained in compounds IV which can react in the Suzuki reaction.

In the phosphorus ligands III, Ar is preferably a C₆-C₁₀-aryl radical, optionally substituted with 1, 2 or 3 substituents independently selected from the group consisting of C₁-C₆-alkyl, C₁-C₆-alkoxy, trifluoromethyl and phenyl. More preferably, Ar is phenyl which may carry 1, 2 or 3 substituents independently selected from the group consisting of C₁-C₆-alkyl, C₁-C₆-alkoxy and trifluoromethyl, and is in particular unsubstituted phenyl.

R³ and R⁴ are preferably, independently of each other, C₁-C₈-alkyl, more preferably branched C₃-C₈-alkyl, and are in particular both tert-butyl.

Salts of the phosphorus ligands are acid addition salts, these thus having the formula: 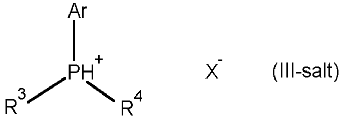 where X- is an anion. Principally any anion derived from a strong acid is suitable, but seeing the desire to avoid certain anions in the waste water, preferred anions are selected from the group consisting of chloride, sulfate, hydrogensulfate, phosphate, hydrogenphosphate, dihydrogenphosphate, perchlorate, tetrafluoroborate, hexafluorophosphate, hydrogenhexafluorozirconate and hydrogenhexafluorotitanate. Specifically, X- is tetrafluoroborate (BF₄⁻).

In particular, the phosphorus ligand III is a compound of the formula IIIa or IIIb in which Ar is phenyl and R³ and R⁴ are both tert-butyl 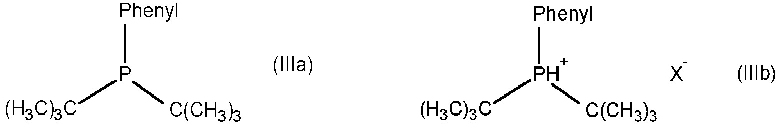 where X⁻ is an anion, preferably selected from the group consisting of chloride, sulfate, hydrogensulfate, phosphate, hydrogenphosphate, dihydrogenphosphate, perchlorate, tetrafluoroborate, hexafluorophosphate, hydrogenhexafluorozirkonate and hydrogenhexafluorotitanate. Specifically, X⁻ is tetrafluoroborate (BF₄⁻).

As said above, the palladium catalyst is introduced into the reaction in the form of a palladium source and a phosphorus ligand of the formula III or a salt thereof, or in form of a palladium complex containing at least one phosphorus ligand of the formula III as defined above or a salt thereof.

If the palladium catalyst is introduced into the reaction in the form of a palladium source and a phosphorus ligand of the formula III or a salt thereof, the complex with the ligand (III) is either formed before the Suzuki reaction starts or, in particular, is formed in situ.

The palladium source is preferably a palladium(II) salt, a palladium complex with ligands different from the ligand of formula III or its salt, or is metallic palladium which is optionally bound to a carrier.

Suitable Pd(ll) salts are for example Pd(ll) acetate, PdCl₂ or Na₂PdCl₄. Preference is given to Pd(ll) acetate and PdCl₂. In particular, Pd(ll) acetate is used.

Suitable Pd(ll) complexes with ligands different from the ligand of formula III or its salt are for example Pd(ll) acetylacetonate or bisacetonitrile Pd(ll) chloride.

A suitable carrier for metallic palladium is charcoal.

The palladium complex containing at least one phosphorus ligand of the formula III as defined above or a salt thereof can be a pre-formed complex of palladium(0) and the ligand III or a salt thereof, or can be a pre-formed palladium(II) complex and the ligand III or a salt thereof.

An example for a palladium complex containing at least one phosphorus ligand of the formula III as defined above or a salt thereof in which palladium is contained as Pd(ll) is Pd(ligand III)₂Cl₂; e.g. dichlorobis(di-(tert-butyl)-phenylphosphine)palladium(II) (Pd(II)[P(C₆H₅)(C(CH₃)₃)₂]₂Cl₂). This complex is commercially available.

An example for a palladium complex containing at least one phosphorus ligand of the formula III as defined above or a salt thereof in which palladium is contained as Pd(0) is Pd(ligand III)₂; e.g. bis(di-(tert-butyl)-phenylphosphine)palladium(0) (Pd(0)[P(C₆H₅)(C(CH₃)₃)₂]₂. This complex is commercially available.

In case that a Pd(ll) salt or a Pd(ll) complex is used, Pd(ll) is reduced to Pd(0) before the Suzuki reaction starts. The reduction generally takes place in situ.

In one preferred embodiment, the palladium catalyst is introduced into the reaction in form of a palladium(II) salt, specifically Pd(ll) acetate or PdCl₂, and the ligand III or a salt thereof.

In another preferred embodiment, the palladium catalyst is introduced into the reaction in form of a pre-formed complex of palladium(0) or (II) and the ligand III or a salt thereof, where the catalyst is specifically selected from the group consisting of bis(di-(tert-butyl)-phenylphosphine)palladium(0) and dichlorobis(di-(tert-butyl)-phenylphosphine)palladium(II).

If the palladium catalyst is not introduced into the reaction in form of the pre-formed complex of palladium and the ligand III, but in form of a Pd source (e.g. a palladium(II) salt, a palladium complex with ligands different from III (or its salt) or a palladium(0) source), and a phosphorus ligand of the formula III or a salt thereof, the Pd source (calculated on the basis of the Pd content) and the ligand of formula III or its salt are used in a molar ratio of preferably from 5:1 to 1:5, more preferably from 2:1 to 1:3, even more preferably from 1.5:1 to 1:2.5, in particular from 1.1:1 to 1:2.5, specifically from 1.05:1 to 1:2.2, very specifically from 1:1 to 1:2.

The Pd catalyst, to be more precise the Pd source or the preformed Pd complex containing at least one phosphorus ligand of the formula III as defined above or a salt thereof (in both cases calculated on the basis of the Pd content) can principally be used in an amount of up to 5 mol%, e.g. of from 0.0001 mol% to 5 mol%, relative to 1 mol of compound II or of compound IV (1 mol of compound II or of compound IV corresponding to 100 mol%). If compounds II and IV are not used in equimolar amounts, the above mol% relate to 1 mol of that compound II or IV which is not used in excess. The ligand III or its salt and the other reaction conditions allow however for the use of Pd in significantly lower amounts. Thus, preferably, the Pd catalyst (calculated on the basis of the Pd content) is used in an amount of from 0.0001 mol% to 0.5 mol%, more preferably from 0.0001 mol% to 0.1 mol%, in particular from 0.0001 mol% to 0.01 mol%, and specifically from 0.001 mol% to 0.007 mol%, very specifically from 0.002 to 0.006 mol%, relative to 1 mol of compound II or of compound IV (1 mol of compound II or of compound IV corresponding to 100 mol%). If compounds II and IV are not used in equimolar amounts, the above mol% relate to 1 mol of that compound II or IV which is not used in excess. Where the amount of the Pd catalyst is related to the compound IV, the latter is of course counted as the number of phenyl rings contained therein which can react in the Suzuki reaction. In other words, where the amount of the Pd catalyst is related to the compound IV, the amount of the Pd catalyst of course actually relates to 1 mol of phenyl rings contained in compound IV which can react in the Suzuki reaction. Thus, for example, in case of borinic acids IVc, which have two phenyl rings, x mol% Pd, relative to 1 mol of compound IVc, means in this case x mol% Pd relative to 1 mol of phenyl rings contained in IVc, and thus to 0.5 mol of compound IVc taken as such. Phenyl rings contained in compound IV which can react in the Suzuki reaction are those phenyl rings which are directly bound to the boron atom.

In case of compounds IV, "equimolar amounts" and "excess" amounts are of course related to the number of phenyl rings contained in compounds IV which can react in the Suzuki reaction.

The reaction is carried out in a solvent mixture of water and an organic solvent which is at least partially miscible with water. "Miscible" means that a homogenous solution is formed. In terms of the present invention, organic solvents which are at least partially miscible with water are solvents which have a miscibility with water of at least 50 g /100 g of water, preferably of at least 100 g /100 g of water, at 20°C.

The solvents are preferably polar aprotic.

Polar aprotic solvents are solvents without a functional group from which a proton can dissociate. Examples for suitable polar aprotic solvents are amides, such as N,N-dimethylformamide (DMF) and N,N-dimethylacetamide; sulfoxides, such as dimethylsulfoxide (DMSO); lactams, such as N-methylpyrrolidone (NMP); cyclic ethers, such as tetrahydrofuran, 1,3-dioxane and 1,4-dioxane; ketones, such as acetone and methylethylketone; nitriles, such as acetonitrile; lactones, such as γ-butyrolactone; nitro compounds, such as nitromethane; ureas, such as tetramethyl urea or dimethylpropylene urea (DMPU); sulfones, such as sulfolan; and carbonic acid esters, such as dimethylcarbonate or ethylenecarbonate. Among the above solvents, preference is given to the cyclic ethers tetrahydrofuran, 1,3-dioxane and 1,4-dioxane. In particular, tetrahydrofuran is used.

In particular the solvent mixture in which the Suzuki reaction is carried out does not contain any other solvent than water and the organic solvent which is at least partially miscible with water.

Preferably, in the solvent mixture water and the organic solvent which is at least partially miscible with water are contained in following amounts:
- water: 0.1 to 80% by weight, based on the total amount of the solvent mixture; and
- organic solvent which is at least partially miscible with water: 20 to 99.9% by weight, based on the total amount of the solvent mixture;
where the amounts of water and organic solvent which is at least partially miscible with water add to 100% by weight.

More preferably, in the solvent mixture water and the organic solvent which is at least partially miscible with water are contained in following amounts:
- water: 0.1 to 70% by weight, based on the total amount of the solvent mixture; and
- organic solvent which is at least partially miscible with water: 30 to 99.9% by weight, based on the total amount of the solvent mixture;
where the amounts of water and organic solvent which is at least partially miscible with water add to 100% by weight.

Even more preferably, in the solvent mixture water and the organic solvent are contained in following amounts:
- water: 5 to 70% by weight, based on the total amount of the solvent mixture; and
- organic solvent which is at least partially miscible with water: 30 to 95% by weight, based on the total amount of the solvent mixture;
where the amounts of water and organic solvent add to 100% by weight.

Particularly preferably, in the solvent mixture water and the organic solvent are contained in following amounts:
- water: 10 to 70% by weight, based on the total amount of the solvent mixture; and
- organic solvent which is at least partially miscible with water: 30 to 90% by weight, based on the total amount of the solvent mixture;
where the amounts of water and organic solvent add to 100% by weight.

In particular, in the solvent mixture water and the organic solvent are contained in following amounts:
- water: 25 to 70% by weight, based on the total amount of the solvent mixture; and
- organic solvent which is at least partially miscible with water: 30 to 75% by weight, based on the total amount of the solvent mixture;
where the amounts of water and organic solvent add to 100% by weight.

Specifically, in the solvent mixture water and the organic solvent are contained in following amounts:
- water: 35 to 70% by weight, based on the total amount of the solvent mixture; and
- organic solvent which is at least partially miscible with water: 30 to 65% by weight, based on the total amount of the solvent mixture;
where the amounts of water and organic solvent add to 100% by weight.

The Suzuki reaction is carried out in the presence of a base. Suitable are both inorganic and organic bases.

Suitable inorganic bases are for example from alkali metal carbonates, e.g. Li₂CO₃, Na₂CO₃, K₂CO₃ or CS₂CO₃, earth alkaline metal carbonates, e.g. MgCO₃ or CaCO₃, alkali metal phosphates, e.g. Li₃PO₄, Na₃PO₄, K₃PO₄ or Cs₃PO₄, earth alkaline metal phosphates, e.g. Mg₃(PO₄)₂ or Ca₃(PO₄)₂, alkali metal hydrogenphosphates, e.g. Li₂HPO₄, Na₂HPO₄, K₂HPO₄ or Cs₂HPO₄, earth alkaline metal hydrogenphosphates, e.g. MgHPO₄ or CaHPO₄, alkali metal hydroxides, LiOH, NaOH or KOH, and earth alkaline metal hydroxides, e.g. Mg(OH)₂ or Ca(OH)₂.

Examples for suitable organic bases are open-chained amines, e.g. trimethylamine, triethylamine, tripropylamine, ethyldiisopropylamine and the like, or basic N-heterocycles, such as morpoline, pyridine, lutidine, DABCO, DBU or DBN.

Preference is however given to inorganic bases, such as to the above alkali metal carbonates, earth alkaline metal carbonates, alkali metal phosphates, earth alkaline metal phosphates, alkali metal hydrogenphosphates, earth alkaline metal hydrogenphosphates, alkali metal hydroxides and earth alkaline metal hydroxides. More preference is given to alkali metal carbonates, alkali metal phosphates and alkali metal hydrogenphosphates. Even more preferred are alkali metal carbonates, such as the above-mentioned Li₂CO₃, Na₂CO₃, K₂CO₃ or Cs₂CO₃. In particular, Na₂CO₃ or K₂CO₃ are used. Specifically, K₂CO₃ is used. In view of corrosive properties of carbonates under certain conditions, it may however be more advantageous to use one of the above-listed phosphates. Thus, in an alternative even more preferred embodiment, alkali metal phosphates, such as the above-mentioned Li₃PO₄, Na₃PO₄, K₃PO₄ or Cs₃PO₄, are used. Specifically, Na₃PO₄ or K₃PO₄ are used.

The base is preferably used in an amount 0.5 to 5 mol per mol of compound II or of compound IV, more preferably from 1 to 4 mol per mol of compound II or of compound IV, in particular from 1 to 3 mol per mol of compound II or of compound IV, specifically from 1 to 2.2 mol per mol of compound II or of compound IV, and very specifically from 1 to 2 mol per mol of compound II or of compound IV. If compounds II and IV are not used in equimolar amounts, the above relation is to 1 mol of that compound II or IV which is not used in excess. Where the amount of the base is related to the compound IV, the latter is of course counted as the number of phenyl rings contained therein which can react in the Suzuki reaction. In other words, where the amount of the base is related to the compound IV, the amount of the base of course actually relates to 1 mol of phenyl rings contained in compound IV which can react in the Suzuki reaction. Thus, for example, in case of borinic acids IVc, which have two phenyl rings, x mol% of base, relative to 1 mol of compound IVc, means in this case x mol% of base relative to 1 mol of phenyl rings contained in IVc, and thus to 0.5 mol of compound IVc taken as such.

As said above, phenyl rings contained in compound IV which can react in the Suzuki reaction are those phenyl rings which are directly bound to the boron atom.

In case of compounds IV, "equimolar amounts" and "excess" amounts are of course related to the number of phenyl rings contained in compounds IV which can react in the Suzuki reaction.

The reaction is preferably carried out at a temperature of from 80 to 120°C; more preferably from 90 to 110°C, in particular of from 95 to 110°C.

The reaction pressure is principally not critical. As however elevated temperatures are used and in case that the solvents used have a boiling point beneath the desired temperature, the reaction is in this case generally carried out in a closed vessel. This results in an inherent pressure, which is generally in the range of from 1.1 to 10 bar (110 to 1000 kPa), in particular from 1.5 to 5 bar (150 to 500 kPa), specifically from 2 to 4 bar (200 to 400 kPa). The exertion of additional pressure, e.g. by pressurizing with an inert gas, is not necessary.

The reaction can be carried out by standard proceedings for Suzuki reactions, e.g. by mixing all reagents, inclusive catalyst or catalyst precursor and ligand, base and the solvent mixture, and reacting them at the desired temperature. Alternatively the reagents can be added gradually, especially in the case of a continuous or semicontinuous process.

The reaction is preferably carried out in an inert atmosphere in order to avoid the presence of oxygen, e.g. under an argon or nitrogen atmosphere.

The reaction is preferably carried out in a pressure vessel, e.g. an autoclave.

After completion of the reaction, the reaction mixture is worked up and the compound of the formula I is isolated in a customary manner. For example, the solvents are removed, for example under reduced pressure. Preferably, however, the work-up is effected by adding water to the reaction mixture, if desired removing the organic solvent which is at least partially miscible with water, e.g. via distillation, if expedient under reduced pressure, adding a non-polar organic solvent and separating the two phases (aqueous and organic phase).

Non-polar organic solvents in terms of the present invention are those which have a miscibility with water of below 20 g /100 g of water at 20°C. Examples are aliphatic hydrocarbons, such as alkanes, e.g. pentane, hexane, heptane, octane, mixtures thereof and technical mixtures, such as petrol ether; cycloaliphatic hydrocarbons, such as cycloalkanes, e.g. cyclohexane, cycloheptane, or cyclooctane; chlorinated aliphatic hydrocarbons, such as halogenalkanes, e.g. dichloromethane, trichloromethane, tetrachloromethane, dichloroethane or tetrachloroethane, aromatic hydrocarbons, such as benzene, toluene, the xylenes, ethylbenzene, cumene (isopropylbenzene), chlorobenzene, o-dichlorobenzene or nitrobenzene, open-chained ethers, such as diethylether, dipropylether, methyl-tert-butylether or methylisobutylether, and higher alkanols, such as n-butanol or isobutanol. Specifically, a higher alkanol is used.

The product I is in the organic phase mainly formed by the non-polar organic solvent. Moreover, the organic phase also contains the Pd catalyst. To enhance the yield, the aqueous phase can be extracted once or more times with an organic solvent, such as the above listed non-polar organic solvents. If desired, the product I can then be separated from the catalyst and optionally from other undesired components, such as unreacted starting compounds II and/or IV, via customary means. For example, the compound I is crystallized from the organic phase. Alternatively, the solvent is removed from the organic phase, e.g. by distillation, e.g. under vacuum, optionally after drying the organic phase, and the solid matter is taken up in another solvent in which the compound I crystallizes better. In yet another alternative, the solid matter is submitted to a chromatographic separation.

Further purification of the product I can be effected if desired; for example by extraction, crystallization, distillation or by chromatography.

If desired, the compound I can then be converted into products of value, such as the below described carboxamides of formula V. Thus, in a further aspect, the invention relates to a process for preparing compounds of the formula V 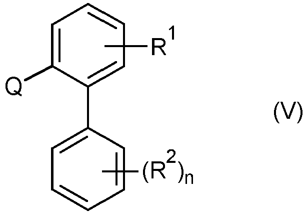 where R¹, R² and n have one of the above general or, in particular, one of the above preferred meanings, and Q is Q¹, Q² or Q³ 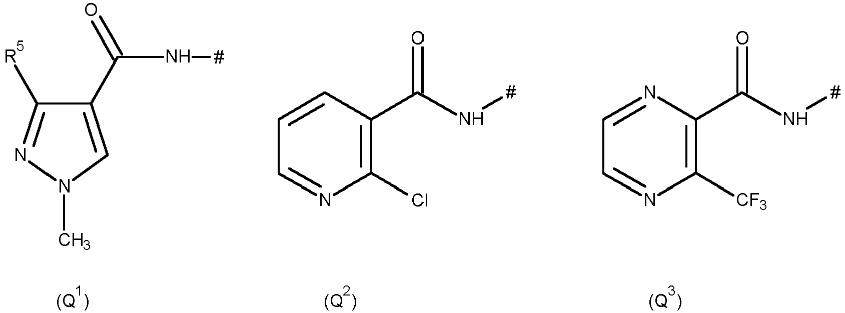 with R⁵ being methyl, optionally substituted by 1, 2 or 3 fluorine atoms, and # being the attachment point to the remainder of the molecule;
which process comprises
(a) preparing a compound of the formula I as defined above with a process as defined above;
(b) reducing the nitro group of the compound of formula I obtained in step (a) to an amino group to obtain a compound of the formula VI 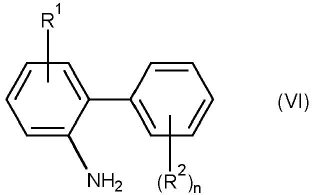 and
(c) reacting the amino compound of the formula VI with a compound Q¹¹, Q²¹ or Q³¹ 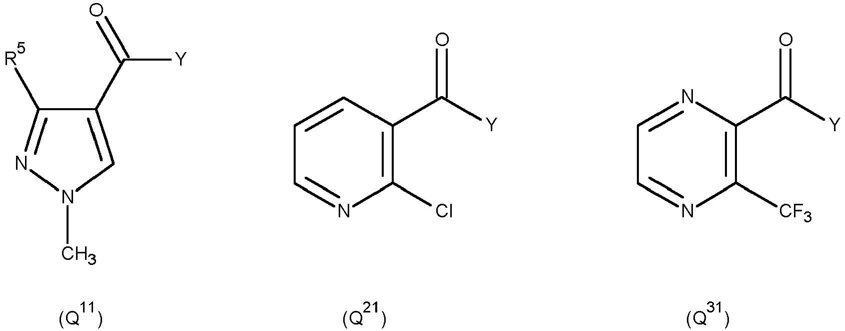
where R⁵ is as defined above and Y is a leaving group.

Reduction in step (b) may be carried out with hydrogen in the presence of a hydrogenation catalyst, such as Pt on charcoal, or with other reduction agents, such as SnCl₂/HCl, Fe/HCI or Fe/NH₄Cl.

Reduction can be carried out according to known methods of converting aromatic nitro compounds into the corresponding aromatic amino compounds, such as described, for example, in R. J. Rahaim, R. E. Maleczka (Jr.), Org. Lett., 2005, 7, 5087-5090, G. S. Vanier, Synlett, 2007, 131-135, S. Chandrasekhar, S. Y. Prakash, C. L. Rao, J. Org. Chem., 2006, 71, 2196-2199, H. Berthold, T. Schotten, H. Hönig, Synthesis, 2002, 1607-1610, and C. Yu, B. Liu, L. Hu, J. Org. Chem., 2001, 66, 919-924.

To obtain compounds V, the amino compound VI is subjected in step (c) to an N-acylation with an acyl precursor Q¹¹, Q²¹ or Q³¹.

Suitable leaving groups Y are -OH, a halide, especially chloride or bromide, -OR^{A}, or -O-C(O)-R^{B}.

If compounds Q¹¹, Q²¹ or Q³¹ are acids, i.e. Y = OH, the reaction can be performed in the presence of a coupling reagent. Suitable coupling reagents (activators) are well known in the art.

If Y = halide, the reaction is expediently performed in the presence of a base. Suitable bases are those listed above in context with the Suzuki coupling.

If Y = OR^{A}, the compounds Q¹¹, Q²¹ or Q³¹ are esters. Suitable esters derive preferably from C₁-C₄-alkanols R^{A}OH in which R^{A} is C₁-C₄-alkyl, or from C₂-C₆-polyols such as glycol, glycerol, trimethylolpropane, erythritol, pentaerythritol and sorbitol. Alternatively, the ester is a so-called active ester, which is obtained in a formal sense by the reaction of the acid Q¹¹, Q²¹ or Q³¹ (Y = OH) with an active ester-forming alcohol, such as p-nitrophenol, N-hydroxybenzotriazole (HOBt), N-hydroxysuccinimide or OPfp (pentafluorophenol).

If compounds Q¹¹, Q²¹ or Q³¹ are anhydrides, i.e. Y = O-C(O)-R^{B}, these are either a symmetric anhydride or an asymmetric anhydride in which -O-OC-R^{B} is a group which can be displaced easily by the 2-aminobiphenyl (VI) used in the reaction. Suitable acid derivatives with which the carboxylic acid Q¹¹, Q²¹ or Q³¹ with Y = OH can form suitable mixed anhydrides are, for example, the esters of chloroformic acid, for example isopropyl chloroformate and isobutyl chloroformate, or of chloroacetic acid.

The acylation can be carried out under known conditions.

The method of the invention yields compounds I in high yields, although an aromatic chloride is used instead of the generally more reactive aromatic bromides or iodides, as used for example in WO 2015/011032. Moreover, the method requires distinctly lower amounts of Pd than most prior art processes. The Suzuki reaction proceeds very selectively, effectively suppressing homocoupling reactions. The process is very well suited for large scale production, and the workup is very simple. Moreover, as the required amounts of Pd are so low, the catalyst does not need to be recycled, which is a very time-consuming and costly procedure, but can be disposed of after the reaction.

The invention is further illustrated by the following examples.

### Examples

### Example 1: Synthesis of 3,4,5-trifluoro-2'-nitrobiphenyl

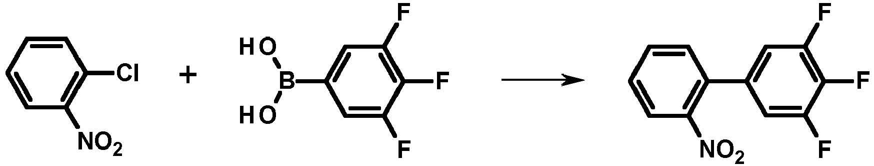

3.9 mg of palladium acetate (0.017 mmol, 0.0024 mol%) and 5.4 mg of di-tert-butyl(phenyl)phosphonium tetrafluoroborate (0.017 mmol) were dissolved in 2 mL of THF at 25°C to form a catalyst solution.

387 g of a potassium carbonate solution (50% in water, 194 g = 1.40 mol of potassium carbonate) were placed in an autoclave. The pressure was reduced to 200 mbar (20 kPa) two times and the autoclave filled with nitrogen. 784 g of a (3,4,5-trifluorophenyl)boronic acid solution (0.70 mol of the boronic acid, 15.7% in THF:water / 3:8; 0.70 mol of the boronic acid) and 212 g of a 1-chloro-2-nitrobenzene solution (50% in THF, 106 g = 0.67 mol of 1-chloro-2-nitrobenzene) were added together with the catalyst solution. 262 g of additional THF were used to transfer the starting materials completely. The reactor was evacuated to 200 mbar (20 kPa) twice and filled with nitrogen. The pressure was reduced a third time to 200 mbar (20 kPa), the autoclave was closed and heated to 110°C outer temperature. Post-stirring was continued for 5 h. The autoclave was cooled to 25°C, the pressure was released and 350 g water were added.

THF was removed by distillation, isobutanol was added and the aqueous phase was removed at 70 °C. The product was crystallized by cooling to -5°C. The product was filtered and washed with water. 174 g of 1,2,3-trifluoro-5-(2-nitrophenyl)benzene (= 3,4,5-trifluoro-2'-nitrobiphenyl) containing 6.8 wt% of water and 1.1 wt% of isobutanol were obtained (calculated yield: 0.63 mol, 94%).

¹H-NMR: (400 MHz, CDCl₃): 7.94 (d, 1H), 7.65 (t, 1H), 7.56 (t, 1H), 7.41 (d, 1H), 6.96 (t,2H) ppm.

### Example 2: Synthesis of 4-chloro-2'-nitrobiphenyl

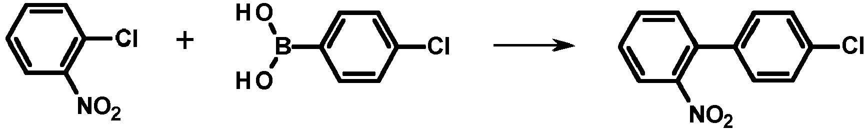

4.5 mg of palladium acetate (0.020 mmol) and 12.4 mg of di-tert-butyl(phenyl)-phosphonium tetrafluoroborate (0.040 mmol) were dissolved in 2.0 g of THF at 25°C to form a catalyst solution.

6.92 g of a potassium carbonate solution (50% in water, 3.46 g = 25 mmol of potassium carbonate), 2.05 g of (4-chlorophenyl)boronic acid (95% purity, 12.5 mmol of the boronic acid) and 2.31 g of a 1-chloro-2-nitrobenzene solution (85% in THF, 1.96 g = 12.5 mmol of 1-chloro-2-nitrobenzene) were placed in an autoclave. 69 mg of the catalyst solution prepared above were added (0.68 µmol Pd, 0.0054 mol%) together with 4.7 g of THF. The pressure was reduced to 200 mbar (20 kPa) twice and the autoclave filled with nitrogen. After reducing the pressure to 200 mbar (20 kPa) the mixture was heated to 100°C. Post-stirring was continued for 5 h, after which the mixture was cooled to 25°C, 6.25 g of water was added and the phases were separated at 50 C. The organic phase was analyzed by HPLC: 86 area-% of the desired product 1-(4-chlorophenyl)-2-nitro-benzene (4 area% remaining boronic acid, 4 area% 1-chloro-2-nitrobenzene).

HPLC-method: Agilent 1050; column: Chromolith RP-18e 100x3 mm; mobile phase: acetonitrile/H₂O 1:1 + 0.5% 0.5 mol/L H₂SO₄; flow 1.0 mL/min; temperature 30°C. 1-(4-chlorophenyl)-2-nitro-benzene (= 4-chloro-2'-nitrobiphenyl): retention time: 3.58 min

### Example 3: Synthesis of 4-chloro-2'-nitrobiphenyl

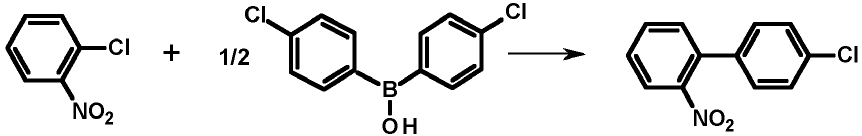

1.37 mg of palladium chloride (0.010 mmol) and 5.27 mg of di-tert-butyl(phenyl)-phosphonium tetrafluoroborate (0.020 mmol) were dissolved in 0.5 g of water and 0.4 g of THF at 25°C to form a catalyst solution.

99.8 g of a potassium carbonate solution (50% in water, 49.9 g = 0.362 mol of potassium carbonate), 189.2 g of a bis(4-chlorophenyl)borinic acid solution (12% in THF:water 95:5, 22.7 g = 0.091 mol of the borinic acid) and 35.6 g of 1-chloro-2-nitrobenzene (used as an 80% solution in THF, 28.5 g = 0.181 mol of 1-chloro-2-nitrobenzene) were placed in an autoclave. 0.885 g of the catalyst solution prepared above were added (0.010 mmol Pd, 0.0055 mol% based on transferred Cl-phenyl; i.e. relative to 0.5 mol of the borinic acid) together with 1.0 g of THF. After three times flushing the autoclave using 3.5 bar (350 kPa) of nitrogen and releasing to 0.5 bar (50 kPa) the mixture was heated to 105°C. Post-stirring was continued for 4 h after which the mixture was cooled to 30°C, 142 g of HCl 10% solution (0.394 mol) were added and the phases were separated at 30 °C. The organic phase was analyzed by HPLC showing complete conversion to the desired product 1-(4-chlorophenyl)-2-nitrobenzene (= 4-chloro-2'-nitrobiphenyl) with no remaining borinic acid.

### Example 4: Synthesis of 3,4,5-trifluoro-2'-nitrobiphenyl using sodium phosphate as a base

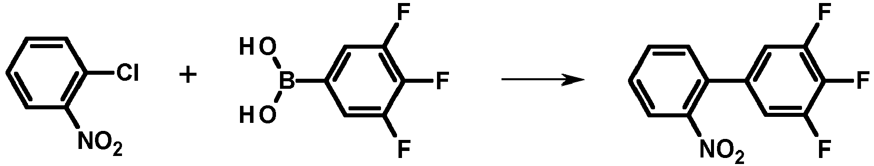

6.2 mg (0.010 mmol, 0.005 mol% relative to 1 mol of the boronic acid) of preformed dichlorobis(di-tert-butylphenylphosphine) palladium(II) was dissolved in 2 mL of THF at 25°C to form a catalyst solution. 34.6 g of phosphoric acid (85%, 300 mmol, 1.5 equivalents relative to the boronic acid) was neutralised with 144.0 g of NaOH (25% in water, 900 mmol) to form a solution of sodium phosphate in an autoclave. The pressure was reduced to 200 mbar (20 kPa) and the autoclave filled with nitrogen. 223.0 g of a (3,4,5-trifluorophenyl)boronic acid solution (200 mmol, 15.8% in THF:water / 3:8) and 64.5 g of a 1-chloro-2-nitrobenzene solution (50% in THF, 32.3 g = 205 mmol of 1-chloro-2-nitrobenzene) were added together with the catalyst solution. 45 g of additional THF were used to transfer the starting materials completely. The reactor was evacuated to 200 mbar (20 kPa) and filled with nitrogen. The pressure was reduced again to 200 mbar (20 kPa), the autoclave was closed and heated to 110°C outer temperature. Post-stirring was continued for 5 h. The autoclave was cooled to 25°C, the pressure was released and the organic phase analysed by quantitative HPLC showing complete conversion to the desired product 3,4,5-trifluoro-2'-nitrobiphenyl with no remaining boronic acid.

### Example 5: Synthesis of 3,4,5-trifluoro-2'-nitrobiphenyl using potassium phosphate as a base

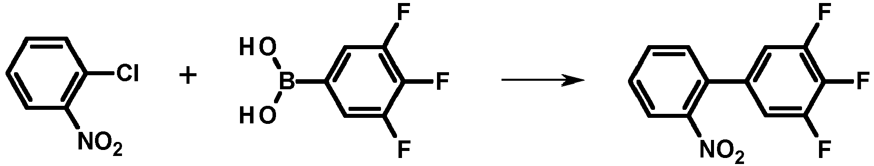

12.4 mg (0.020 mmol, 0.005 mol% relative to 1 mol of the boronic acid) preformed dichlorobis(di-tert-butylphenylphosphine) palladium(II) was dissolved in 2 mL of THF at 25°C to form a catalyst solution. 97.0 g of potassium phosphate monohydrate (95%, 400 mmol, 1.0 equivalents relative to the boronic acid) was dissolved in 215 g of water and placed in an autoclave. The pressure was reduced to 200 mbar (20 kPa) and the autoclave filled with nitrogen. 446.0 g of a (3,4,5-trifluorophenyl)boronic acid solution (400 mmol, 15.8% in THF:water / 3:8) and 128.6 g of a 1-chloro-2-nitrobenzene solution (50% in THF, 64.3 g = 407 mmol of 1-chloro-2-nitrobenzene) were added together with the catalyst solution. 90 g of additional THF were used to transfer the starting materials completely. The reactor was evacuated to 200 mbar (20 kPa) and filled with nitrogen. The pressure was reduced again to 200 mbar (20 kPa), the autoclave was closed and heated to 110°C outer temperature. Post-stirring was continued for 5 h. The autoclave was cooled to 25°C, the pressure was released and the organic phase analysed by quantitative HPLC showing complete conversion to the desired product 3,4,5-trifluoro-2'-nitrobiphenyl with no remaining boronic acid.

## Claims

1. A process for preparing substituted biphenyl compounds of the formula I 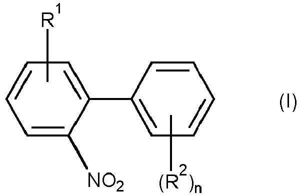 in which the substituents are each defined as follows:
R¹ is hydrogen, cyano, F, C₁-C₄-alkyl, C₁-C₄-haloalkyl, C₁-C₄-alkoxy or C₁-C₄-haloalkoxy;
R² is cyano, nitro, F, Cl, C₁-C₄-alkyl, C₁-C₄-haloalkyl, C₃-C₁₀-cycloalkyl which may carry 1, 2, 3 or 4 C₁-C₄-alkyl substituents; C₃-C₁₀-halocycloalkyl, C₁-C₆-alkoxy, C₁-C₆-haloalkoxy, C₁-C₆-alkylcarbonyl, C₁-C₆-haloalkylcarbonyl, C₁-C₆-alkoxycarbonyl, or C₁-C₆-haloalkoxycarbonyl; and
n is 0, 1, 2 or 3, where, in case that n is 2 or 3, the R² radicals may have identical or different definitions;
which comprises reacting a compound of the formula II 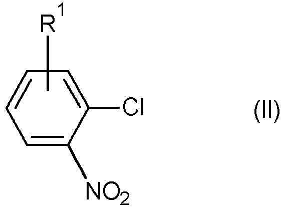 in which R¹ is as defined above,
in the presence of a base and of a palladium catalyst,
where the palladium catalyst is introduced into the reaction in the form of
- a palladium source and a phosphorus ligand of the formula III or a salt thereof 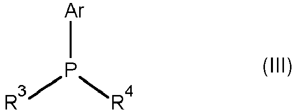 in which
Ar is a C₆-C₁₀-aryl radical or a 5- or 6-membered heteroaryl ring containing 1, 2, 3 or 4 heteroatoms selected from the group consisting of N and O as ring members, where the aryl radical and the heteroaryl ring are optionally substituted with 1, 2 or 3 substituents independently selected from the group consisting of C₁-C₆-alkyl, C₁-C₆-alkoxy, trifluoromethyl and phenyl;
R³ is C₁-C₈-alkyl or C₃-C₁₀-cycloalkyl; and
R⁴ is C₁-C₈-alkyl or C₃-C₁₀-cycloalkyl;
or
- a palladium complex containing at least one phosphorus ligand of the formula III as defined above or a salt thereof;
in a solvent mixture of water and an organic solvent which is at least partially miscible with water,
with an organoboron compound of the formula IV 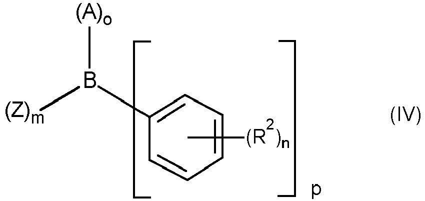 wherein R² and n are as defined above and the compound of formula IV is selected from the group consisting of
(i) boronic acids with o = 0, m = 2; p = 1 and Z = hydroxyl groups, or their trimers;
(ii) boronic acid derivates with o = 0, m = 2; p = 1 and Z = halogen; C₁-C₄-alkyl, C₁-C₆-alkoxy or C₆-C₁₀-aryloxy;
(iii) borinic acids or borinic acid derivatives with o = 0, m = 1; p = 2 and Z = hydroxy, halogen, C₁-C₄-alkyl, C₁-C₆-alkoxy or C₆-C₁₀-aryloxy;
(iv) mixed borinic acids or borinic acid derivatives with o = 1, m = 1; p = 1, A = C₁-C₄-alkyl and Z = hydroxy, halogen, C₁-C₄-alkyl, C₁-C₆-alkoxy or C₆-C₁₀-aryloxy;
(v) cyclic boronic esters with o = 0, m = 2 and p = 1, wherein the two Z groups form together a bridging group -O-(CH₂)_{q}-O-, wherein q is 2 or 3, so that the two Z groups, together with the boron atom to which they are attached, form a 5- or 6- membered ring, where the CH₂ groups are optionally substituted by one or two C₁-C₄-alkyl groups;
(vi) boronates with o = 0, m = 3, p = 1 and Z = hydroxyl, halogen, C₁-C₄-alkyl, C₁-C₆-alkoxy or C₆-C₁₀-aryloxy, and accompanied by a cation which compensates the negative charge of the boronate anion;
(vii) triarylboranes with o = 0, m = 0 and p = 3;
(viii) tetraarylborates with o = 0, m = 0 and p = 4, and accompanied by a cation which compensates the negative charge of the borate anion;
where the reaction is carried out at a temperature of from 80 to 140°C.

2. The process according to claim 1, wherein R¹ is hydrogen or fluorine.

3. The process according to any of the preceding claims, wherein R² is fluorine or chlorine, and n is 1, 2 or 3.

4. The process according to any of the preceding claims, wherein the biphenyl compound I is 4-chloro-2'-nitro-biphenyl, 3,4-dichloro-2'-nitro-biphenyl, 3,4-difluoro-2'-nitro-biphenyl, 3,4,5-trifluoro-2'-nitro-biphenyl, 3-chloro-4,5-difluoro-2'-nitro-biphenyl, 3,4-dichloro-5'-fluoro-2'-nitro-biphenyl or 3,5-dichloro-4-fluoro-2'-nitro-biphenyl.

5. The process according to any of the preceding claims, wherein the phosphorus ligand of the formula III is a compound of the formula IIIa or IIIb 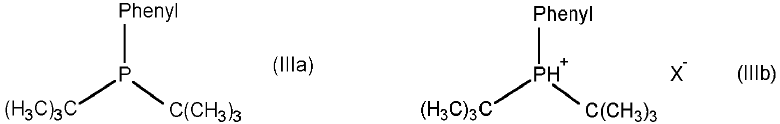 where X⁻ means an anion.

6. The process according to any of the preceding claims, wherein the palladium catalyst is introduced into the reaction in the form of a palladium source and a phosphorus ligand of the formula III or a salt thereof, wherein the palladium source is a palladium(II) salt, or is a palladium complex with ligands different from the ligand of formula III or its salt, or is metallic palladium which is optionally bound to a carrier; or wherein the palladium catalyst is introduced into the reaction in the form of a palladium complex selected from the group consisting of bis(di-(tert-butyl)-phenylphosphine)palladium(0) and dichlorobis(di-(tert-butyl)-phenylphosphine)palladium(II).

7. The process according to any of the preceding claims, wherein the palladium catalyst, calculated on the basis of the Pd content, is used in an amount of from 0.0001 mol% to 0.5 mol%, in particular of from 0.0001 mol% to 0.1 mol%, relative to 1 mol of compound II or of compound IV (the latter counted as the number of phenyl rings contained therein which can react in the Suzuki reaction), if these are used in equimolar amounts, or, if compounds II and IV are not used in equimolar amounts, relative to 1 mol of that compound II or IV (the latter counted as the number of phenyl rings contained therein which can react in the Suzuki reaction) which is not used in excess.

8. The process according to claim 7, wherein the palladium catalyst, calculated on the basis of the Pd content, is used in an amount of from 0.0001 mol% to 0.01 mol%, preferably from 0.001 mol% to 0.01 mol%, in particular from 0.001 to 0.007 mol%, specifically from 0.002 to 0.006 mol%, relative to 1 mol of compound II or of compound IV (the latter counted as the number of phenyl rings contained therein which can react in the Suzuki reaction), if these are used in equimolar amounts, or, if compounds II and IV are not used in equimolar amounts, relative to 1 mol of that compound II or IV (the latter counted as the number of phenyl rings contained therein which can react in the Suzuki reaction) which is not used in excess.

9. The process according to any of the preceding claims, wherein the organoboron compound IV is a phenylboronic acid IVa or a diphenylborinic acid IVc, 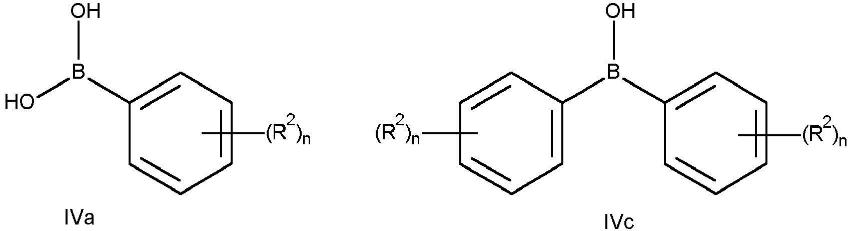 or a mixture of IVa and IVc, in which R² and n are each as defined in any of claims 1 or 3; and is in particular a phenylboronic acid IVa.

10. The process according to any of the preceding claims, wherein the reaction is performed at a temperature of from 80 to 120°C; more preferably from 90 to 110°C, in particular of from 95 to 110°C.

11. The process according to any of the preceding claims, wherein the organic solvent which is at least partially miscible with water is a cyclic ether, in particular tetrahydrofuran.

12. The process according to any of the preceding claims, where in the solvent mixture water and the organic solvent which is at least partially miscible with water are contained in following amounts:
- water: 0.1 to 80% by weight, based on the total amount of the solvent mixture; and
- organic solvent which is at least partially miscible with water: 20 to 99.9% by weight, based on the total amount of the solvent mixture;
where the amounts of water and organic solvent which is at least partially miscible with water add to 100% by weight;
and where in particular in the solvent mixture water and the organic solvent which is at least partially miscible with water are contained in following amounts:
- water: 0.1 to 70% by weight, based on the total amount of the solvent mixture; and
- organic solvent which is at least partially miscible with water: 30 to 99.9% by weight, based on the total amount of the solvent mixture;
where the amounts of water and organic solvent which is at least partially miscible with water add to 100% by weight.

13. The process according to claim 12, where in the solvent mixture water and the organic solvent which is at least partially miscible with water are contained in following amounts:
- water: 25 to 70% by weight, based on the total amount of the solvent mixture; and
- organic solvent which is at least partially miscible with water: 30 to 75% by weight, based on the total amount of the solvent mixture;
where the amounts of water and organic solvent which is at least partially miscible with water add to 100% by weight.

14. The process according to any of the preceding claims, wherein the base is selected from inorganic bases, in particular from alkali metal carbonates, earth alkaline metal carbonates, alkali metal phosphates, earth alkaline metal phosphates, alkali metal hydrogenphosphates, earth alkaline metal hydrogenphosphates, alkali metal hydroxides and earth alkaline metal hydroxides.

15. A process for preparing compounds of the formula V 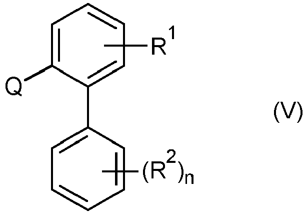 where R¹, R² and n are as defined in any of claims 1 to 4 and Q is Q¹, Q² or Q³ 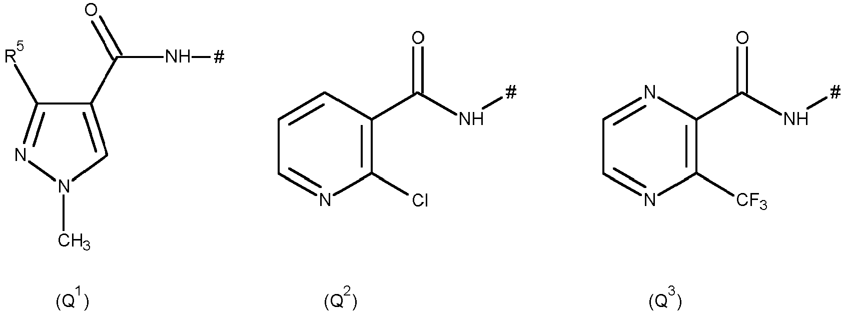 with R⁵ being methyl, optionally substituted by 1, 2 or 3 fluorine atoms, and
# being the attachment point to the remainder of the molecule;
which process comprises
(a) preparing a compound of the formula I as defined in any of claims 1 to 4 with a process according to any of the preceding claims;
(b) reducing the nitro group of the compound of formula I obtained in step (a) to an amino group to obtain a compound of the formula VI 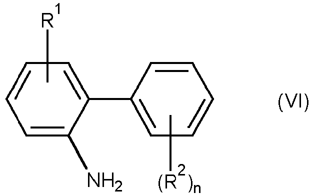 and
(c) reacting the amino compound of the formula VI with a compound Q¹¹, Q²¹ or Q³¹ 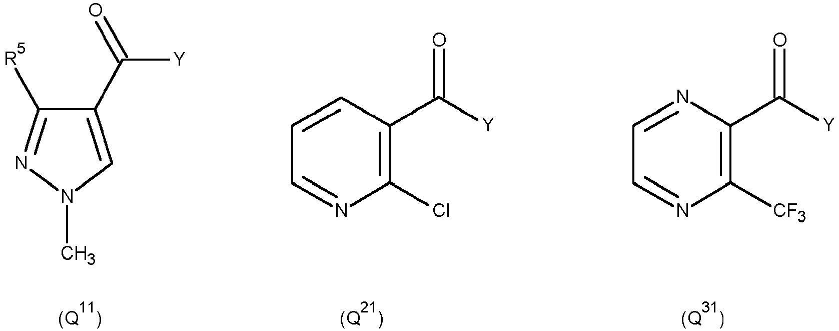
where R⁵ is as defined above and Y is a leaving group.

## Patentansprüche

1. Verfahren zur Herstellung substituierter Biphenylverbindungen der Formel I 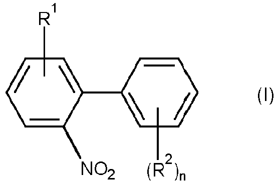 in der die Substituenten folgende Bedeutungen haben:
R¹ Wasserstoff, Cyano, F, C₁-C₄-Alkyl, C₁-C₄-Halogenalkyl, C₁-C₄-Alkoxy oder C₁-C₄-Halogenalkoxy;
R² Cyano, Nitro, F, Cl, C₁-C₄-Alkyl, C₁-C₄-Halogen-alkyl, C₃-C₁₀-Cycloalkyl, das 1, 2, 3 oder 4 C₁-C₄-Alkylsubstituenten tragen kann, C₃-C₁₀-Halogencycloalkyl, C₁-C₆-Alkoxy, C₁-C₆-Halogen-alkoxy, C₁-C₆-Alkylcarbonyl, C₁-C₆-Halogenalkyl-carbonyl, C₁-C₆-Alkoxycarbonyl oder C₁-C₆-Halogenalkoxycarbonyl; und
n 0, 1, 2 oder 3, wobei im Fall von n gleich 2 oder 3 die Reste R² gleiche oder verschiedene Bedeutungen haben können;
bei dem man eine Verbindung der Formel II 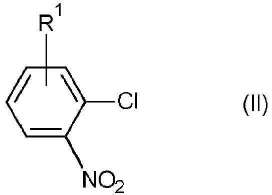 in der R¹ wie oben definiert ist,
in Gegenwart einer Base und eines Palladiumkatalysators,
wobei der Palladiumkatalysator in Form
- einer Palladiumquelle und eines Phosphorliganden der Formel III oder eines Salzes davon 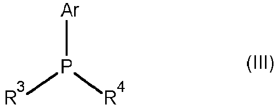 wobei
Ar für einen C₆-C₁₀-Arylrest oder einen 5- 6-gliedrigen Heteroarylring mit 1, 2, 3 oder 4 Heteroatomen aus der Gruppe bestehend aus N und O als Ringgliedern steht, wobei der Arylrest und der Heteroarylring gegebenenfalls durch 1, 2 oder 3 Substituenten, die unabhängig aus der Gruppe bestehend aus C₁-C₆-Alkyl, C₁-C₆-Alkoxy, Trifluormethyl und Phenyl ausgewählt sind, substituiert sind;
R³ für C₁-C₈-Alkyl oder C₃-C₁₀-Cycloalkyl steht und
R⁴ für C₁-C₈-Alkyl oder C₃-C₁₀-Cycloalkyl steht;
- eines Palladiumkomplexes, der mindestens einen Phosphorliganden der Formel II gemäß obiger Definition oder ein Salz davon enthält;
in die Umsetzung eingetragen wird;
in einem Lösungsmittelgemisch aus Wasser und einem organischen Lösungsmittel, das zumindest teilweise mit Wasser mischbar ist,
mit einer Organoborverbindung der Formel IV 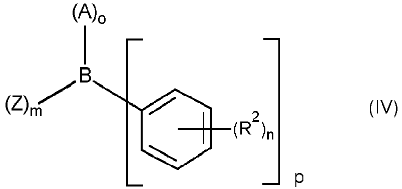 wobei R² und n wie oben definiert sind und die Verbindung der Formel IV aus der Gruppe bestehend aus
(i) Boronsäuren mit o = 0, m = 2; p = 1 und Z = Hydroxylgruppen oder deren Trimeren;
(ii) Boronsäurederivaten mit o = 0, m = 2; p = 1 und Z = Halogen, C₁-C₄-Alkyl, C₁-C₆-Alkoxy oder C₆-C₁₀-Aryloxy;
(iii) Borinsäuren bzw. Borinsäurederivaten mit o = 0, m = 1; p = 2 und Z = Hydroxy, Halogen, C₁-C₄-Alkyl, C₁-C₆-Alkoxy oder C₆-C₁₀-Aryloxy;
(iv) gemischten Borinsäuren bzw. Borinsäurederivaten mit o = 1, m = 1; p = 1, A = C₁-C₄-Alkyl und Z = Hydroxy, Halogen, C₁-C₄-Alkyl, C₁ -C₆-Al koxy oder C₆-C₁₀-Aryloxy;
(v) cyclischen Boronsäureestern mit o = 0, m = 2 und b = 1, wobei die beiden Z-Gruppen zusammen eine Brückengruppe -O-(CH₂)_{q}-O-bilden, wobei q für 2 oder 3 steht, so dass die beiden Z-Gruppen zusammen mit dem Boratom, an das sie gebunden sind, einen 5- oder 6-gliedrigen Ring bilden, wobei die CH₂-Gruppen gegebenenfalls durch eine oder zwei C₁-C₄-Alkylgruppen substituiert sind;
(vi) Boronaten mit o = 0, m = 3; p = 1 und Z = Hydroxyl, Halogen, C₁-C₄-Alkyl, C₁-C₆-Alkoxy oder C₆-C₁₀-Aryloxy und in Begleitung eines Kations, das die negative Ladung des Boronat-Anions ausgleicht;
(vii) Triarylboranen mit o = 0, m = 0 und p = 3;
(viii) Tetraarylboraten mit o = 0, m = 0 und p = 4 ausgewählt wird; und in Begleitung eines Kations, das die negative Ladung des Boronat-Anions ausgleicht;
umsetzt;
wobei die Umsetzung bei einer Temperatur von 80 bis 140 °C durchgeführt wird.

2. Verfahren nach Anspruch 1, bei dem R¹ für Wasserstoff oder Fluor steht.

3. Verfahren nach einem der vorhergehenden Ansprüche, bei dem R² für Fluor oder Chlor steht und n für 1, 2 oder 3 steht.

4. Verfahren nach einem der vorhergehenden Ansprüche, bei dem es sich bei der Biphenylverbindung I um 4-Chlor-2'-nitrobiphenyl, 3,4-Dichlor-2'-nitrobiphenyl, 3,4-Difluor-2'-nitrobiphenyl, 3,4,5-Trifluor-2'-nitrobiphenyl, 3-Chlor-4,5-difluor-2'-nitrobiphenyl, 3,4-Dichlor-5'-fluor-2'-nitrobiphenyl oder 3,5-Dichlor-4-fluor-2'-nitrobiphenyl handelt.

5. Verfahren nach einem der vorhergehenden Ansprüche, bei dem es sich bei dem Phosphorliganden der Formel III um eine Verbindung der Formel IIIa oder IIIb handelt: 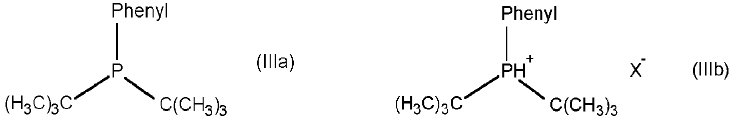 wobei X⁻ ein Anion bedeutet.

6. Verfahren nach einem der vorhergehenden Ansprüche, bei dem der Palladiumkatalysator in Form einer Palladiumquelle und eines Phosphorliganden der Formel III oder eines Salzes davon in die Umsetzung eingetragen wird, wobei es sich bei der Palladiumquelle um ein Palladium(II)-Salz oder einen Palladiumkomplex mit Liganden, die von dem Liganden der Formel III oder dessen Salz verschieden sind, oder um gegebenenfalls an einen Träger gebundenes metallisches Palladium handelt; oder bei dem der Palladiumkatalysator in Form eines Palladiumkomplexes aus der Gruppe bestehend aus Bis(di-(tert-butyl)phenylphosphin)palladium(0) und Dichlorobis(di-(tert-butyl)phenylphosphin)-palladium(II) in die Umsetzung eingetragen wird.

7. Verfahren nach einem der vorhergehenden Ansprüche, bei dem der Palladiumkatalysator, berechnet auf der Basis des Pd-Gehalts, in einer Menge von 0,0001 Mol-% bis 0,5 Mol-%, insbesondere von 0,0001 Mol-% bis 0,1 Mol-%, bezogen auf 1 mol Verbindung II oder Verbindung IV (wobei Letztere als Zahl der darin enthaltenen Phenylringe, die in der Suzuki-Reaktion reagieren können, gezählt wird), wenn diese in äquimolaren Mengen verwendet werden, oder dann, wenn die Verbindungen II und IV nicht in äquimolaren Mengen verwendet werden, bezogen auf 1 mol der Verbindung II oder IV (wobei Letztere als Zahl der darin enthaltenen Phenylringe, die in der Suzuki-Reaktion reagieren können, gezählt wird), die nicht im Überschuss verwendet wird, verwendet wird.

8. Verfahren nach Anspruch 7, bei dem der Palladiumkatalysator, berechnet auf der Basis des Pd-Gehalts, in einer Menge von 0,0001 Mol-% bis 0,01-%, insbesondere von 0,001 Mol-% bis 0,01 Mol-%, insbesondere von 0,001 bis 0,007 Mol-%, speziell von 0,002 bis 0,006 Mol-%, bezogen auf 1 mol Verbindung II oder Verbindung IV (wobei Letztere als Zahl der darin enthaltenen Phenylringe, die in der Suzuki-Reaktion reagieren können, gezählt wird), wenn diese in äquimolaren Mengen verwendet werden, oder dann, wenn die Verbindungen II und IV nicht in äquimolaren Mengen verwendet werden, bezogen auf 1 mol der Verbindung II oder IV (wobei Letztere als Zahl der darin enthaltenen Phenylringe, die in der Suzuki-Reaktion reagieren können, gezählt wird), die nicht im Überschuss verwendet wird, verwendet wird.

9. Verfahren nach einem der vorhergehenden Ansprüche, bei dem es sich bei der Organoborverbindung IV um eine Phenylboronsäure IVa oder eine Diphenylborinsäure IVc 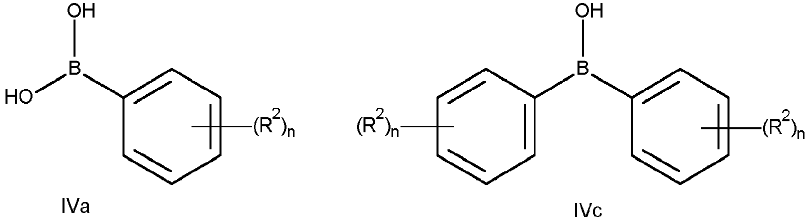 oder eine Mischung von IVa und IVc, wobei R² und n jeweils wie in einem der Ansprüche 1 oder 3 definiert sind; und insbesondere um eine Phenylboronsäure IVa handelt.

10. Verfahren nach einem der vorhergehenden Ansprüche, bei dem die Umsetzung bei einer Temperatur von 80 bis 120 °C, weiter bevorzugt von 90 bis 110 °C, insbesondere von 95 bis 110 °C, durchgeführt wird.

11. Verfahren nach einem der vorhergehenden Ansprüche, bei dem es sich bei dem organischen Lösungsmittel, das zumindest teilweise mit Wasser mischbar ist, um einen cyclischen Ether, insbesondere Tetrahydrofuran, handelt.

12. Verfahren nach einem der vorhergehenden Ansprüche, wobei in dem Lösungsmittelgemisch Wasser und das organische Lösungsmittel, das zumindest teilweise mit Wasser mischbar ist, in den folgenden Mengen enthalten sind:
- Wasser: 0,1 bis 80 Gew.-%, bezogen auf die Gesamtmenge des Lösungsmittelgemischs; und
- organisches Lösungsmittel, das zumindest teilweise mit Wasser mischbar ist: 20 bis 99,9 Gew.-%, bezogen auf die Gesamtmenge des Lösungsmittelgemischs;
wobei sich die Mengen von Wasser und organischem Lösungsmittel, das zumindest teilweise mit Wasser mischbar ist, zu 100 Gew.-% summieren;
und wobei insbesondere in dem Lösungsmittelgemisch Wasser und das organische Lösungsmittel, das zumindest teilweise mit Wasser mischbar ist, in den folgenden Mengen enthalten sind:
- Wasser: 0,1 bis 70 Gew.-%, bezogen auf die Gesamtmenge des Lösungsmittelgemischs; und
- organisches Lösungsmittel, das zumindest teilweise mit Wasser mischbar ist: 30 bis 99,9 Gew.-%, bezogen auf die Gesamtmenge des Lösungsmittelgemischs;
wobei sich die Mengen von Wasser und organischem Lösungsmittel, das zumindest teilweise mit Wasser mischbar ist, zu 100 Gew.-% summieren.

13. Verfahren nach Anspruch 12, wobei in dem Lösungsmittelgemisch Wasser und das organische Lösungsmittel, das zumindest teilweise mit Wasser mischbar ist, in den folgenden Mengen enthalten sind:
- Wasser: 25 bis 70 Gew.-%, bezogen auf die Gesamtmenge des Lösungsmittelgemischs; und
- organisches Lösungsmittel, das zumindest teilweise mit Wasser mischbar ist: 30 bis 75 Gew.-%, bezogen auf die Gesamtmenge des Lösungsmittelgemischs;
wobei sich die Mengen von Wasser und organischem Lösungsmittel, das zumindest teilweise mit Wasser mischbar ist, zu 100 Gew.-% summieren.

14. Verfahren nach einem der vorhergehenden Ansprüche, bei dem die Base aus anorganischen Basen, insbesondere aus Alkalimetallcarbonaten, Erdalkalimetallcarbonaten, Alkalimetallphosphaten, Erdalkalimetallphosphaten, Alkalimetallhydrogenphosphaten, Erdalkalimetallhydrogenphosphaten, Alkalimetallhydroxiden und Erdalkalimetallhydroxiden, ausgewählt wird.

15. Verfahren zur Herstellung von Verbindungen der Formel V 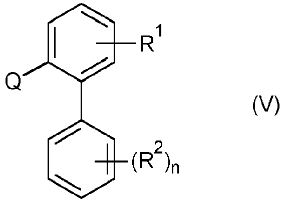 wobei R¹, R² und n wie in einem der Ansprüche 1 bis 4 definiert sind und Q für Q¹, Q² oder Q³ 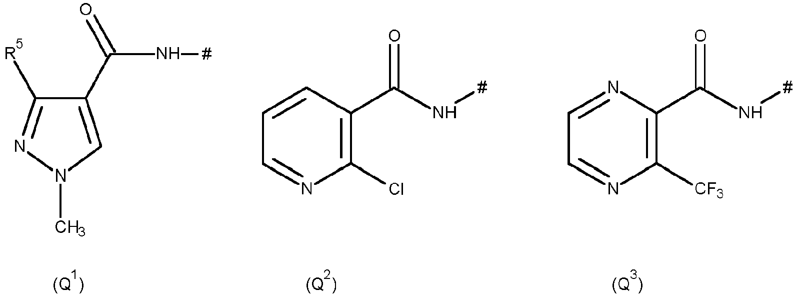 steht, wobei R⁵ für Methyl, das gegebenenfalls durch 1, 2 oder 3 Fluoratome substituiert ist, steht und # der Verknüpfungspunkt mit dem Rest des Moleküls ist;
bei dem man
(a) mit einem Verfahren gemäß einem der vorhergehenden Ansprüche eine Verbindung der Formel I gemäß einem der Ansprüche 1 bis 4 herstellt;
(b) die Nitrogruppe der in Schritt (a) erhaltenen Verbindung der Formel I zu einer Aminogruppe reduziert, wobei man eine Verbindung der Formel VI 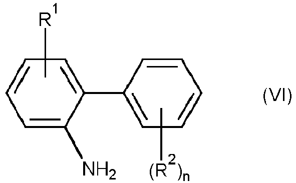 erhält;
und
(c) die Aminoverbindung der Formel VI mit einer Verbindung Q¹¹, Q²¹ oder Q³¹ 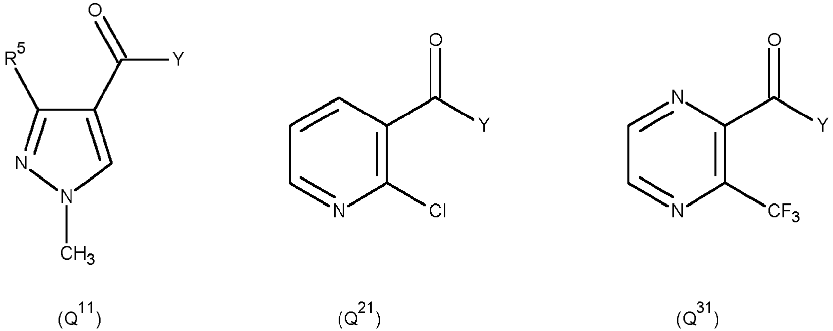 wobei R⁵ wie oben definiert ist und Y für eine Abgangsgruppe steht, umsetzt.

## Revendications

1. Procédé de préparation de composés de biphényle substitués de formule I 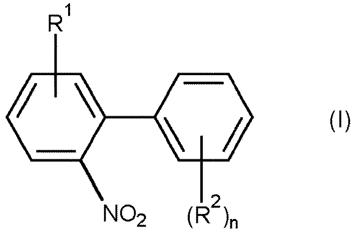 dans laquelle les substituants sont chacun définis comme suit :
R¹ est hydrogène, cyano, F, alkyle en C₁-C₄, halogénoalkyle en C₁-C₄, alcoxy en C₁-C₄ ou halogénoalcoxy en C₁-C₄ ;
R² est cyano, nitro, F, Cl, alkyle en C₁-C₄, halogénoalkyle en C₁-C₄, cycloalkyle en C₃-C₁₀ qui peut porter 1, 2, 3 ou 4 substituants alkyle en C₁-C₄ ; halogénocycloalkyle en C₃-C₁₀, alcoxy en C₁-C₆, halogénoalcoxy en C₁-C₆, (alkyle en C₁-C₆) carbonyle, (halogénoalkyle en C₁-C₆) carbonyle, (alcoxy en C₁-C₆) carbonyle, ou (halogénoalcoxy en C₁-C₆) carbonyle ; et
n est 0, 1, 2 ou 3, où, dans le cas où n est 2 ou 3, les radicaux R² peuvent avoir des définitions identiques ou différentes ;
qui comprend la réaction d'un composé de formule II 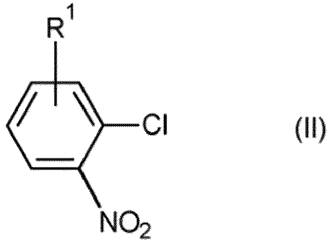
dans laquelle R¹ est tel que défini ci-dessus, en présence d'une base et d'un catalyseur au palladium, où le catalyseur au palladium est introduit dans la réaction sous la forme de
- une source de palladium et un ligand de phosphore de formule III ou un sel de celui-ci 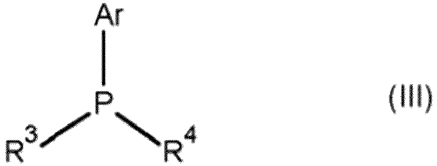 dans laquelle
Ar est un radical aryle en C₆-C₁₀ ou un cycle hétéroaryle de 5 ou 6 chaînons contenant 1, 2, 3 ou 4 hétéroatomes choisis dans le groupe constitué de N et O en tant que chaînons de cycle, où le radical aryle et le cycle hétéroaryle sont facultativement substitués par 1, 2 ou 3 substituants indépendamment choisis dans le groupe constitué d'alkyle en C₁-C₆, alcoxy en C₁-C₆, trifluorométhyle et phényle ;
R³ est alkyle en C₁-C₈ ou cycloalkyle en C₃-C₁₀ ; et
R⁴ est alkyle en C₁-C₈ ou cycloalkyle en C₃-C₁₀ ;
ou
- un complexe de palladium contenant au moins un ligand de phosphore de formule III tel que défini ci-dessus ou un sel de celui-ci ;
dans un mélange de solvants d'eau et d'un solvant organique qui est au moins partiellement miscible dans l'eau,
avec un composé organoboré de formule IV 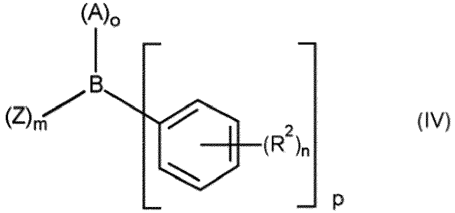 dans lequel R² et n sont tels que définis ci-dessus et le composé de formule IV est choisi dans le groupe constitué de
(i) acides boroniques avec o = 0, m = 2 ; p = 1 et Z = groupes hydroxyle, ou leurs trimères ;
(ii) dérivés d'acide boronique avec o = 0, m = 2 ; p = 1 et Z = halogène ; alkyle en C₁-C₄, alcoxy en C₁-C₆ ou aryloxy en C₆-C₁₀ ;
(iii) acides boriniques ou dérivés d'acide borinique avec o = 0, m = 1 ; p = 2 et Z = hydroxy, halogène, alkyle en C₁-C₄, alcoxy en C₁-C₆ ou aryloxy en C₆-C₁₀ ;
(iv) acides boriniques ou dérivés d'acide borinique mixtes avec o = 1, m = 1 ; p = 1, A = alkyle en C₁-C₄ et Z = hydroxy, halogène, alkyle en C₁-C₄, alcoxy en C₁-C₆ ou aryloxy en C₆-C₁₀ ;
(v) esters boroniques cycliques avec o = 0, m = 2 et p = 1, dans lesquels les deux groupes Z forment conjointement un groupe de pontage -O-(CH₂)_{q}-O-, dans lequel q est 2 ou 3, de sorte que les deux groupes Z, conjointement avec l'atome de bore auquel ils sont liés, forment un cycle de 5 ou 6 chaînons, où les groupes CH₂ sont facultativement substitués par un ou deux groupes alkyle en C₁-C₄ ;
(vi) boronates avec o = 0, m = 3, p = 1 et Z = hydroxyle, halogène, alkyle en C₁-C₄, alcoxy en C₁-C₆ ou aryloxy en C₆-C₁₀ et accompagnés d'un cation qui compense la charge négative de l'anion borate ;
(vii) triarylboranes avec o = 0, m = 0 et p = 3 ;
(viii) tétraarylborates avec o = 0, m = 0 et p = 4, et accompagnés d'un cation qui compense la charge négative de l'anion borate ;
où la réaction est conduite à une température de 80 à 140 °C.

2. Procédé selon la revendication 1, dans lequel R¹ est hydrogène ou fluor.

3. Procédé selon l'une quelconque des revendications précédentes, dans lequel R² est fluor ou chlore, et n est 1, 2 ou 3.

4. Procédé selon l'une quelconque des revendications précédentes, dans lequel le composé de biphényle I est 4-chloro-2'-nitro-biphényle, 3,4-dichloro-2'-nitro-biphényle, 3,4-difluoro-2'-nitro-biphényle, 3,4,5-trifluoro-2'-nitro-biphényle, 3-chloro-4,5-difluoro-2'-nitro-biphényle, 3,4-dichloro-5'-fluoro-2'-nitro-biphényle ou 3,5-dichloro-4-fluoro-2'-nitro-biphényle.

5. Procédé selon l'une quelconque des revendications précédentes, dans lequel le ligand de phosphore de formule III est un composé de formule IIIa ou IIIb 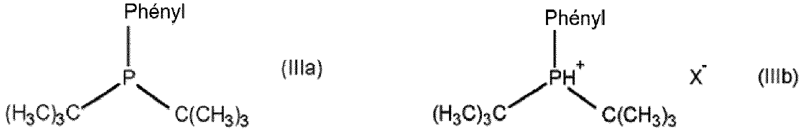 où X- désigne un anion.

6. Procédé selon l'une quelconque des revendications précédentes, dans lequel le catalyseur au palladium est introduit dans la réaction sous la forme d'une source de palladium et d'un ligand de phosphore de formule III ou un sel de celui-ci, dans lequel la source de palladium est un sel de palladium(II), ou est un complexe de palladium avec des ligands différents du ligand de formule III ou son sel, ou est du palladium métallique qui est facultativement lié à un support ; ou dans lequel le catalyseur au palladium est introduit dans la réaction sous la forme d'un complexe de palladium choisi dans le groupe constitué de bis(di-(tert-butyl)-phénylphosphine)palladium(0) et dichlorobis(di-(tert-butyl)-phénylphosphine)palladium(II).

7. Procédé selon l'une quelconque des revendications précédentes, dans lequel le catalyseur au palladium, calculé sur la base de la teneur en Pd, est utilisé en une quantité de 0, 0001 % en moles à 0,5 % en moles, en particulier de 0, 0001 % en moles à 0, 1 % en moles, par rapport à 1 mol de composé II ou de composé IV (ce dernier étant compté comme étant le nombre de cycles phényle contenus dans celui-ci qui peuvent réagir dans la réaction de Suzuki), si ceux-ci sont utilisés dans des quantités équimolaires, ou, si les composés II et IV ne sont pas utilisés dans des quantités equimolaires, par rapport à 1 mol de ce composé II ou IV (ce dernier étant compté comme étant le nombre de cycles phényle contenus dans celui-ci qui peuvent réagir dans la réaction de Suzuki) qui n'est pas utilisé en excès.

8. Procédé selon la revendication 7, dans lequel le catalyseur au palladium, calculé sur la base de la teneur en Pd, est utilisé en une quantité de 0,0001 % en moles à 0,01 % en moles, de préférence de 0,001 % en moles à 0,01 % en moles, en particulier de 0,001 à 0,007 % en moles, spécifiquement de 0,002 à 0,006 % en moles, par rapport à 1 mol de composé II ou de composé IV (ce dernier étant compté comme étant le nombre de cycles phényle contenus dans celui-ci qui peuvent réagir dans la réaction de Suzuki), si ceux-ci sont utilisé dans des quantités equimolaires, ou, si les composés II et IV ne sont pas utilisés dans des quantités equimolaires, par rapport à 1 mol de ce composé II ou IV (ce dernier étant compté comme étant le nombre de cycles phényle contenus dans celui-ci qui peuvent réagir dans la réaction de Suzuki) qui n'est pas utilisé en excès.

9. Procédé selon l'une quelconque des revendications précédentes, dans lequel le composé organoboré IV est un acide phénylboronique IVa ou un acide diphénylborinique IVc, 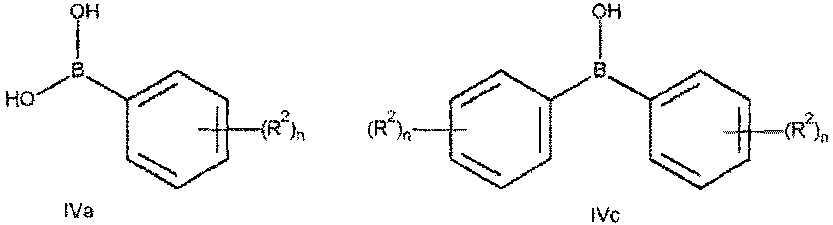 ou un mélange de IVa et IVc, dans lesquels R² et n sont chacun tels que définis dans l'une quelconque des revendications 1 ou 3 ; et sont en particulier l'acide phénylboronique IVa.

10. Procédé selon l'une quelconque des revendications précédentes, dans lequel la réaction est conduite à une température de 80 à 120 °C ; plus préférablement de 90 à 110 °C, en particulier de 95 à 110 °C.

11. Procédé selon l'une quelconque des revendications précédentes, dans lequel le solvant organique qui est au moins partiellement miscible dans l'eau est un éther cyclique, en particulier le tétrahydrofurane.

12. Procédé selon l'une quelconque des revendications précédentes, où, dans le mélange de solvants, l'eau et le solvant organique qui est au moins partiellement miscible dans l'eau sont contenus dans les quantités suivantes :
- eau : 0, 1 à 80 % en poids, sur la base de la quantité totale du mélange de solvants ; et
- solvant organique qui est au moins partiellement miscible dans l'eau : 20 à 99,9 % en poids, sur la base de la quantité totale du mélange de solvants ;
où la somme des quantités d'eau et de solvant organique qui est au moins partiellement miscible dans l'eau est de 100 % en poids ;
et où, en particulier, dans le mélange de solvants, l'eau et le solvant organique qui est au moins partiellement miscible dans l'eau sont contenus dans les quantités suivantes :
- eau : 0, 1 à 70 % en poids, sur la base de la quantité totale du mélange de solvants ; et
- solvant organique qui est au moins partiellement miscible dans l'eau : 30 à 99,9 % en poids, sur la base de la quantité totale du mélange de solvants ;
où la somme des quantités d'eau et de solvant organique qui est au moins partiellement miscible dans l'eau est de 100 % en poids.

13. Procédé selon la revendication 12, où, dans le mélange de solvants, l'eau et le solvant organique qui est au moins partiellement miscible dans l'eau sont contenus dans les quantités suivantes :
- eau : 25 à 70 % en poids, sur la base de la quantité totale du mélange de solvants ; et
- solvant organique qui est au moins partiellement miscible dans l'eau : 30 à 75 % en poids, sur la base de la quantité totale du mélange de solvants ;
où la somme des quantités d'eau et de solvant organique qui est au moins partiellement miscible dans l'eau est de 100 % en poids.

14. Procédé selon l'une quelconque des revendications précédentes, dans lequel la base est choisie parmi des bases inorganiques, en particulier parmi des carbonates de métal alcalin, des carbonates de métal alcalino-terreux, des phosphates de métal alcalin, des phosphates de métal alcalino-terreux, des hydrogénophosphates de métal alcalin, des hydrogénophosphates de métal alcalino-terreux, des hydroxydes de métal alcalin et des hydroxydes de métal alcalino-terreux.

15. Procédé de préparation de composés de formule V 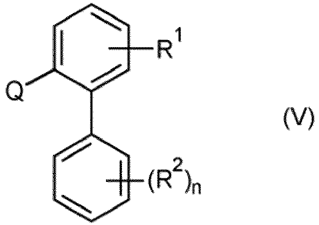 où R¹, R² et n sont tels que définis dans l'une quelconque des revendications 1 à 4 et Q est Q¹, Q² ou Q³ 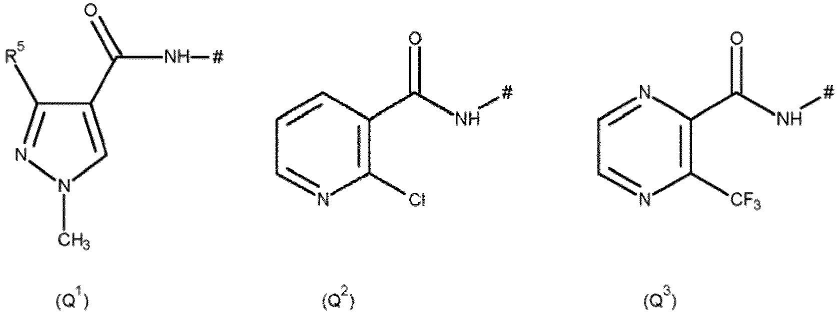 avec R⁵ étant méthyle, facultativement substitué par 1, 2 ou 3 atomes de fluor, et
# étant le point de liaison au reste de la molécule ; ledit procédé comprenant
(a) la préparation d'un composé de formule I tel que défini dans l'une quelconque des revendications 1 à 4 par un procédé selon l'une quelconque des revendications précédentes ;
(b) la réduction du groupe nitro du composé de formule I obtenu dans l'étape (a) en groupe amino pour obtenir un composé de formule VI 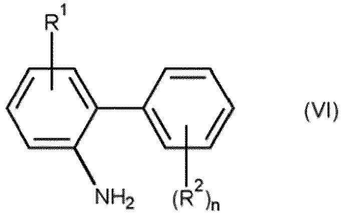 et
(c) la réaction du composé amino de formule VI avec un composé Q¹¹, Q²¹ ou Q³¹ 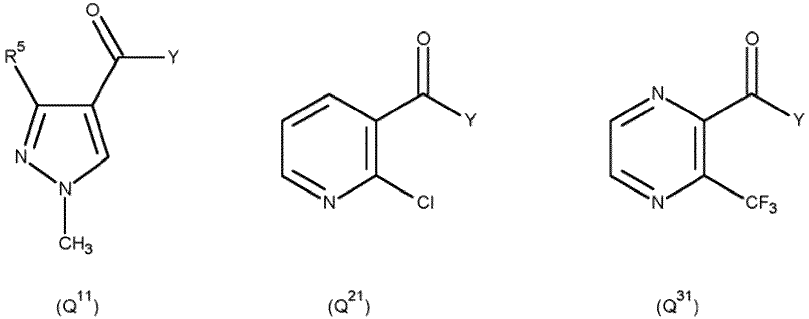 où R⁵ est tel que défini ci-dessus et Y est un groupe partant.
